# EUROPEAN PATENT SPECIFICATION

(11) **EP 4 556 514 B1**
(45) Date of publication and mention of the grant of the patent: **26.08.2026**
(21) Application number: 23755115.5
(22) Date of filing: 13.07.2023
(51) Int. Cl.: C08J 11/14, C08J 11/18, C08G 18/40, C08G 71/04, C08J 11/22, C08J 11/28, B01J 21/02, B01J 31/02

(54) **METHOD FOR POLYURETHANE DEPOLYMERIZATION**
METHODE ZUR POLYURETHANDEPOLYMERISIERUNG
PROCÉDÉ DE DÉPOLYMÉRISATION DE POLYURÉTHANE

(30) Priority: 14.07.2022 ES 202230649
(43) Date of publication of application: 21.05.2025
(73) Proprietor: Universidad de Murcia, 30003 Murcia (ES); Universitat Jaume I, 12071 Castellón De La Plana (ES); Asociación Empresarial Centro Technológico Del Mueble y la Madera de la Región de Murcia, 30510 Yecla Murcia (ES)
(72) Inventor: LOZANO RODRÍGUEZ, Pedro, 30100 Murcia (ES); VILLA AROCA, Rocío, 30100 Murcia (ES); SALAS VIDAL, Rebeca, 30100 Murcia (ES); GARCÍA-VERDUGO CEPEDA, Eduardo, 12071 Castelló de la Plana (CASTELLON) (ES); MACIÁ DELGADO, María, 12071 Castelló de la Plana (CASTELLON) (ES)
(74) Representative: TRBL Intellectual Property
(86) International application number: PCT/ES2023/070452
(87) International publication number: WO 2024/013423

(56) References cited:
- CN-B- 113 717 434
- JP-A- 2003 064 218
- US-A- 5 208 379
- US-A1- 2021 230 391

## Description

### FIELD OF THE INVENTION

The present invention lies within the technical field of chemical recovery of polymers. More specifically, the invention relates to a method for polyurethane depolymerization based on mild reaction conditions and on the use of an active reaction medium, stable over time and completely recoverable and reusable, whereby obtaining a polyol mass readily separable from the reaction medium that does not require a later step of purification for being reused in the synthesis of a new polyurethane.

### BACKGROUND OF THE INVENTION

Polyurethane is a polymer which is obtained by means of the reaction between polyols and diisocyanates, giving rise to the formation of urethane (-NH-CO-O-) and urea (-NH-CO-NH-) bonds if polymerization is carried out in the presence of controlled amounts of water. By means of spectroscopy FT-IR, said urethane bonds are identified selectively and unequivocally by a band between 1700-1740 cm⁻¹ [L. Zhao, V. Semetey. ACS Omega, 2021, 6, p. 4175-4183]. According to their chemical structure, polyurethanes are classified into two large groups, differenced due to their behavior against temperature: thermoplastics, capable of melting and flowing with temperature before degrading; and thermosets, which maintain their shape and strength under high pressure and temperature conditions. The excellent properties of this latter group, which include polyurethane foams, make polyurethane one of the most widely used polymers for manufacturing thermal insulation and consumer products, such as mattresses, seats, furniture, or sponges for cleaning. In 2017, 22.9 million tons of this polymer were produced, increasing since then at an annual rate of 4.5%.

As a result of this growing demand, 10% of the total production of polyurethane is estimated to correspond with removed post-production products and post-production waste, which are disposed of in dumps or incinerated given the absence of profitable and easy-to-implement methods of reuse. This is due, in large part, to the fact that thermoset polyurethanes cannot be melted to be remodeled into other products. However, the permanent needs for space to accumulate waste and the increase in the costs of dumps, along with the hardening of environmental regulations and the greater awareness of society to improve the sustainability of the current way of life, determine the need to develop and implement alternatives to the disposal of polyurethane waste, preferably by means of reincorporating it into the chain of consumption.

The molecular depolymerization of polyurethane waste is one of the strategies of greater interest today for the implementation of circular economy-based industrial processes. In said process, polyurethane waste is transformed into the compounds that gave rise to their synthesis; that is, polyols and diamines from diisocyanates, for the purpose of being reincorporated into a new polyurethane synthesis process, thereby promoting the economic and environmental advantages characteristic of sustainable development and the circular economy [Kemona, A., Piotrowska, M. Polymers, 2020, 12(8), p. 1752].

The most common chemical methods in the depolymerization of polyurethane are hydrolysis and glycolysis, although strategies combining both have also been defined [Gerlock J. et al. (1984). Ind. Eng. Chem. Process Des. Dev., 23(3), p. 545-552]. In the depolymerization by hydrolysis, water is the compound used both as a urethane bond-breaking agent and as a solvent; while, in the case of depolymerization by glycolysis, an alcohol (e.g., methanol) or a glycol (e.g., ethylene glycol, propylene glycol, diethylene glycol) is used in an amount by weight greater than the weight of the polyurethane to be treated (typically 1.5:1 or higher) for both functions.

To date, all the methods for polyurethane depolymerization described in the state of the art have been based on the suspension of a polyurethane mass in an excess of a protic molecular solvent; that is, water, glycol, alcohol, or a mixture thereof, acting as a nucleophilic agent, accompanied or not by a volatile organic cosolvent (typically, toluene or tetrahydrofuran, THF), and in the presence of a carbene type organic catalyst, quaternary ammonium salts, or a conventional base such as NaOH, KOH, etc. (in concentrations greater than 4 N), which have a low catalytic activity, making it necessary to perform said methods at high temperatures (130-250°C) and long reaction times (>12 h), obtaining yields of less than 85% and favoring unwanted side reactions [see, for example, EP0011662B1, US5208379A, WO2010/130652A2; US2004127720A1; EP1693409A1; WO 2022/042909 A1; Gadhave, R.V., et al. Open J. Polym. Chem., 2019, 9 (2), p. 39-51].

Alternatively, the depolymerization of polyurethane foam by means of glycolysis with diols (e.g., ethylene glycol) is a widely described process which also requires drastic reaction conditions [Simón, D. et al. Waste Management, 2018, 16, p. 147-171]. By means of this strategy, polyurethane waste is suspended in an excess of polyol (e.g., ethylene glycol) in the presence of a catalyst (e.g., naphthenic acid, potassium acetate, titanium butoxide, stannous octoate, etc.) for 6-48 h and at temperatures comprised between 150-280°C. This process generates a two-phase system, where the more dense, lower phase contains the polyurethane glycolysis products, whereas the higher phase contains the excess of polyol used as the reaction medium. Furthermore, after the catalytic reaction a later expensive step for the elimination of the excess of glycol by means of vacuum distillation (100 mbar, 170-180°C) is required, for the purpose of achieving a product with a KOH value suitable for a possible reuse in the synthesis of a new polyurethane [see EP1693409A1, JP2003064218A].

In the same sense, glycolysis processes with diethanolamine or ethanolamine have also been described, observing an efficient depolymerization of polyurethane at temperatures close to 200°C using tin salts (e.g., dibutyltin dilaurate, etc.) as a catalyst [see dos Santos, L.M. et al. Polimeros: Ciencia e Tecnologia, 2013, 23, 608-613; Jehanno, C. et al. Polymer Chemistry, 2019, 10, 172-186]. CN113717434B discloses a method for recycling polyurethane foam employing an acidic ionic liquid with high catalytic activity. This patent, however, does not disclosure use of an ionic liquid in combination with a superbase as basic catalyst, nor the use of a protic solvent is to precipitate the polyol mass from the reaction mixture. US2021/230391A1 discloses a depolymerization method of plastic by using of a super-base which can involve an ionic liquid solvent and that can involve the use of a nucleophile to depolymerize polyurethane. However, the method involves a much higher reaction temperature (≥ 150 °C) and does not disclose the use of an antisolvent-related work-up.

Based on this state of the art, the need arises to develop a rapid and efficient method for polyurethane depolymerization based on mild reaction conditions and on the use of an active reaction medium, stable over time and fully recoverable and reusable, whereby obtaining a recycled polyol mass, readily separable from the reaction medium, and that does not require a later step of purification for being reused in the synthesis of a new polyurethane.

### BRIEF DESCRIPTION OF THE INVENTION

The object of the present invention relates to a method for depolymerizing a polyurethane mass, wherein said method comprises performing the following steps:
a) mixing the polyurethane mass with a depolymerization reaction medium, wherein said depolymerization reaction medium comprises an ionic liquid, a basic catalyst and a nucleophilic agent; and
b) obtaining two immiscible fractions of the mixture obtained in step a):
   - a first fraction, comprising at least a polyol mass; and
   - a second fraction, comprising at least the ionic liquid and the basic catalyst.

Advantageously, in the method of the invention:
- the depolymerization reaction medium comprises:
   ▪ an ionic liquid in a proportion equal to or greater than 50% w/w with respect to the total weight of the depolymerization reaction medium;
   ▪ a superbase as a basic catalyst; and
   ▪ a nucleophilic agent in a proportion equal to or less than 15% w/w with respect to the total weight of the depolymerization reaction medium;
- step a) is carried out at a temperature between 50-100°C for a time period comprised between 2 minutes and 10 hours; and
- step b) comprises performing the following sub-steps:
   i. adding a mass of a protic molecular solvent in an amount between 4 and 40 times more the weight of the depolymerization reaction medium to the mixture of step a);
   ii. subjecting the mixture of step i) to a temperature between 20-60°C for at least 1 hour; and
   iii. separating the fractions obtained.

Within the scope of interpretation of the present invention, "ionic liquid" will be understood as any chemical substance formed exclusively by ions and having a melting point below 100°C. Among its physicochemical properties, its virtually zero volatility stands out, which converts compounds of this type in a non-contaminant and reusable alternative to volatile organic solvents. Furthermore, they have excellent heat stability, large capacity to dissolve a broad range of molecular compounds, including gases, and a density, melting point, polarity, and miscibility with water or other molecular solvents, which can be modulated depending on the type of anion and cation forming same. Likewise, "superbase" will be understood as any organic compound with a high proton affinity which contains two or more basic functional groups and one or more nitrogen atoms (N) forming C-N, C=N, C=N-C, - N-H and/or =N-H bonds (where C is the chemical symbol for carbon and H is for hydrogen), and which, when added to a reaction, increases the rate thereof. More preferably, "superbase" shall be understood to mean any amidine, amidine derivative, guanidine, or guanidine derivative, and even more preferably, a bicyclic amidine, a bicyclic amidine derivative, guanidine, or a guanidine derivative with a pKₐ equal to or greater than 12. The term "nucleophilic agent" shall be understood to mean any chemical compound that interacts or reacts with the carbon of the carbonyl group of the urethane bond, causing the rupture of said bond. More preferably, "nucleophilic agent" shall be understood to mean water, an alcohol, a primary amine, a secondary amine, or any possible combination thereof. Lastly, the term "protic molecular solvent" shall be understood as any polar solvent made up of isolated molecules capable of forming hydrogen bonds with other electronegative molecules, donating and accepting protons, with a boiling temperature equal to or less than 100°C and in which the polyol mass recovered by means of the method of the invention is insoluble.

The method of the invention represents a sustainable alternative to the methods for polyurethane depolymerization known to date. First, said method allows the depolymerization of polyurethane at temperatures less than 100°C and with reaction times below 10 h, which simplifies operational systems and facilitates the control and safety of the process. Secondly, the process is characterized by the simultaneous use of an ionic liquid and of a superbase to form the depolymerization reaction medium. The ionic liquid is the major component of said reaction medium, and its use instead of a volatile organic solvent or a glycol offers not only a better conditioning of polyurethane for depolymerization, but also a solution to the problems of solvents emission (due to their low volatility) and waste generation (due to its easy recovery and reuse without the need for expensive purification processes). In turn, the superbase provides a much higher catalytic activity in non-aqueous reaction media and/or with low water content, and a higher stability over time compared to conventional basic catalysts (e.g., NaOH, KOH, carbenes, amines), allowing an easy recovery for reuse. And finally, since the process is performed at temperatures less than 100°C, it can be carried out at atmospheric pressure using a nucleophilic agent with a low boiling point (e.g., water, alcohols such as methanol or ethanol, or primary amines such as butylamine), which determines the absence of a later purification step of the polyol mass resulting from the depolymerization process. These conditions are clearly different from what occurs with conventional glycolysis processes, where the low efficiency of the catalysts determines that high reaction temperatures (130-280°C) are to be used. This makes it necessary to use glycols as they have boiling points close to or greater than 200°C, to reduce risks, but it is necessary to separate the excess of glycol to achieve a polyol that can be reused in the synthesis of new polyurethanes. Furthermore, the use in the method of the invention of a nucleophilic agent with a low boiling point at a concentration equal to or less than 15% w/w, allows its easy, complete removal, since said amount is virtually consumed during the depolymerization reaction of the polyurethane mass. The final possible excess thereof may be readily removed by evaporation or by washing with a protic molecular solvent, preferably water, achieving separation of the polyol mass from the reaction medium.

In a preferred embodiment of the invention, the polyurethane mass comprises a thermoset polyurethane and, more preferably, a polyurethane foam.

In another preferred embodiment of the invention, the polyurethane mass is mixed with the depolymerization reaction medium in a mass ratio comprised between 1:20 and 1:1 in step a); more preferably, between 1:15 and 1:3, and, even more preferably, between 1:10 and 1:5.

In another preferred embodiment of the invention, step a) is carried out at a temperature comprised between 80-100°C and/or for a time period comprised between 2 and 8 hours. More preferably, at a temperature comprised between 90-98°C and/or for a time period comprised between 3 and 7 hours.

In another preferred embodiment of the invention, step a) is carried out in a depolymerization reaction medium comprising an ionic liquid in a proportion between 50-85% w/w with respect to the total weight of the depolymerization reaction medium. More preferably, between 65-80% w/w.

In another preferred embodiment of the invention, step a) is carried out in a depolymerization reaction medium comprising a nucleophilic agent in a proportion between 1-15% w/w with respect to the total weight of the depolymerization reaction medium. More preferably, between 5-15% w/w.

In another preferred embodiment of the invention, in step b) a mass of a protic molecular solvent is added in an amount between 5 and 15 times more the weight of the depolymerization reaction medium.

In another preferred embodiment of the invention, step a) is carried out with a nucleophilic agent comprising water, an alcohol, an amine, or any possible combination thereof. More preferably, the nucleophilic agent comprises at least 80% water due to its higher nucleophilic capacity and low boiling point.

In a preferred embodiment of the invention, sub-step i) is carried out with a protic molecular solvent comprising water, methanol, ethanol, isopropanol, or any possible combination thereof. More preferably, the protic molecular solvent comprises at least 80% water.

In another preferred embodiment of the invention, step a) is carried out with an ionic liquid comprising:
- a cation selected from dialkylimidazolium, tetraalkylammonium, dialkylpiperidinium, alkylpyridinium, dialkylpyrrolidonium, and/or tetraalkylphosphonium; and/or
- an anion selected from fluoride, chloride, bromide, iodide, acetate, trifluoroacetate, formate, and/or carbonate.

More preferably, the ionic liquid comprises 1-butyl-3-methylimidazolium acetate, 1-ethyl-3-methylimidazolium acetate, 1-butyl-3-methylpyridinium chloride, 1-butyl-3-methylpyridinium bromide, 1-butyl-3-methylpyridinium iodide, 1-butyl-1-methylpiperidinium chloride, 1-butyl-1-methylpiperidinium bromide, 1-butyl-1-methylpiperidinium iodide, 1-butyl-3-methylimidazolium chloride, 1-butyl-3-methylimidazolium bromide, 1-butyl-3-methylimidazolium iodide, 1-ethyl-3-methylimidazolium chloride, 1-ethyl-3-methylimidazolium bromide, 1-ethyl-3-methylimidazolium iodide, 1-butyl-4-methylpyridinium chloride, 1-butyl-3-methylimidazolium formate, 1-hexyl-3-methylimidazolium chloride, 1-hexyl-3-methylimidazolium bromide, 1-hexyl-3-methylimidazolium iodide, 1-octyl-3-methylimidazolium chloride, 1-octyl-3-methylimidazolium bromide, 1-octyl-3-methylimidazolium iodide, 1-allyl-3-methylimidazolium chloride, 1-butyl-3-methyl-imidazolium acetate, 1-butyl-3-methylimidazolium bromide, 1-ethyl-2-methylpyridinium bromide, N-ethylpyridinium bromide, 1-butyl-3-methyl-imidazolium chloride, 1-butyl-1-methylpyrrolidinium chloride, 1-butyl-1-methylpiperidinium iodide, tetrabutylphosphonium bromide, tetrabutylphosphonium chloride, 1-butyl-2-methylpyridinium chloride, 1-ethyl-1-methyl-piperidinium chloride, or any possible combination thereof.

In another preferred embodiment of the invention, step a) is carried out with a superbase comprising at least a bicyclic amidine, a bicyclic amidine derivative, a guanidine, a guanidine derivative, or any possible combination thereof. More preferably, the superbase used in the method of the invention comprises 1,8-diazabicyclo [5.4.0] undec-7-ene (DBU), 1,5-diazabicyclo [4.3.0] non-5-ene (DBN), 1,5,7-triazabicyclo [4.4.0] dec-5-ene (TBD), tetramethylguanidine (TMG), or any possible combination thereof.

In another preferred embodiment of the invention, said method comprises performing at least one of the following additional steps:
- reusing the first fraction in the synthesis of a new polyurethane; and/or
- reusing the second fraction in step a) of a new process for depolymerization of a polyurethane mass.

In another preferred embodiment of the invention, said method comprises an additional step of drying the first and/or second fraction until reducing the water content of said fraction/s to a value of less than 1% w/w. In the scope of interpretation of the present invention, "drying" will be understood as any physical or chemical operation that allows the removal of water; preferably, a process of lyophilization, vacuum evaporation, heating, distillation, resin adsorption, or any possible combination thereof.

Additionally, the two preferred embodiments above can comprise a prior step of treating the second fraction with a hydrophobic adsorbent, preferably activated carbon or polystyrene resin.

In another preferred embodiment of the invention, sub-step iii) comprises a process of centrifugation, decantation, filtration, vacuum filtration, resin adsorption, or any possible combination thereof.

In another preferred embodiment of the invention, the method of the invention comprises an additional step of verification of the progression and completion of step a) and/or b). Preferably, said additional step comprises at least one of the following sub-steps:
- taking homogeneous samples of the reaction medium (e.g., 0.2 mL) during the course of step a) at different time intervals and suspending them in a 10 times higher volume of an aprotic polar solvent capable of solubilizing the polymeric polyol (e.g., acetone, dimethyl sulfoxide (DMSO), or tetrahydrofuran), resulting in a solution free of suspended solids when step a) is fully completed;
- performing an analysis by Attenuated Total Reflectance combined with Fourier Transform Infrared Spectroscopy (ATR-FTIR) of the first dry fraction, to observe the absence of the characteristic band of the urethane bond (1700 cm⁻¹-1740 cm⁻¹); and/or
- determining the hydroxyl value (mg KOH/g of sample) by titration of the first dry fraction, with phthalic anhydride/pyridine according to the ASTM D-4274-16 (2016) standard, to quantify the presence of hydroxyl groups in the solid fraction recovered. Since polyols contain multiple hydroxyl groups, the higher said value is, the greater the amount of polyols present in said fraction, demonstrating the correct depolymerization of the polyurethane mass according to the method of the invention.

### DESCRIPTION OF THE FIGURES

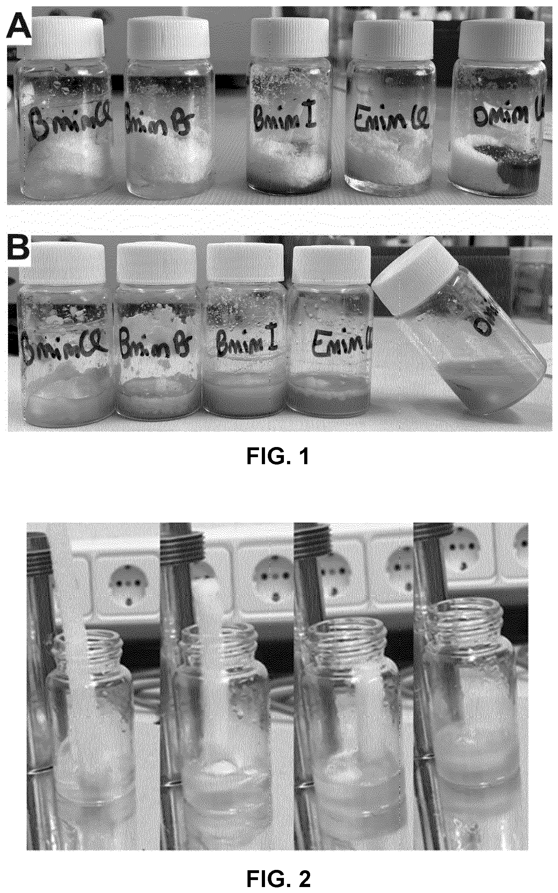

Figure 1 shows five depolymerization reaction mixtures according to preferred embodiments of the invention, containing different ionic liquids at 65% w/w, 20% w/w ground polyurethane foam, 10% w/w 1,8-diazabicyclo [5.4.0] undec-7-ene (DBU), and 5% w/w water, at the beginning of the reaction (Figure 1A), and after 4 hours of incubation with magnetic stirring at 95°C (Figure 1B).
Figure 2 shows the evolution over time of the depolymerization of a polyurethane rod in contact with a depolymerization reaction medium containing 65% w/w 1-butyl-3-methylimidazolium chloride ([Bmim][Cl]), 20% w/w DBU and 15% w/w water, for 2 min at 95°C.

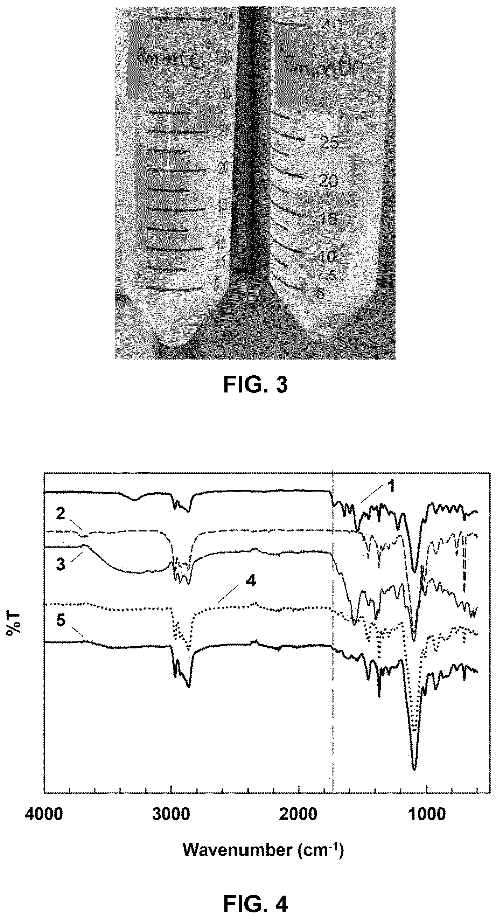

Figure 3 shows the result of the separation by centrifugation of the fractions obtained after the depolymerization of a polyurethane foam mass according to a preferred embodiment of the invention: 95°C for 4 hours in a reaction medium comprising [Bmim][CI], DBU and water (left); and 95°C for 4 hours in a reaction medium comprising 1-butyl-3-methylimidazolium bromide ([Bmim][Br]), DBU and water (right).
Figure 4 shows the ATR-FTIR spectra of: (1) a ground thermoset polyurethane foam mass; (2) a commercial polyol mass for industrial use; (3) a polyol mass recovered by means of the method of the invention using a depolymerization reaction medium comprising 65% w/w 1-butyl-3-methyl-imidazolium acetate ([Bmim][Ac]), 20% w/w DBU, and 15% w/w water; (4) a polyol mass recovered by means of the method of the invention using a depolymerization reaction medium comprising 65% w/w [Bmim][CI], 20% w/w DBU, and 15% w/w water; and (5) a polyol mass recovered by means of the method of the invention using a depolymerization reaction medium comprising 65% w/w 1-ethyl-3-methylimidazolium chloride ([Emim][Cl]), 20% w/w DBU, and 15% w/w water. The characteristic band of the urethane bond (1720-1740 cm⁻¹) is indicated with a dashed vertical line.

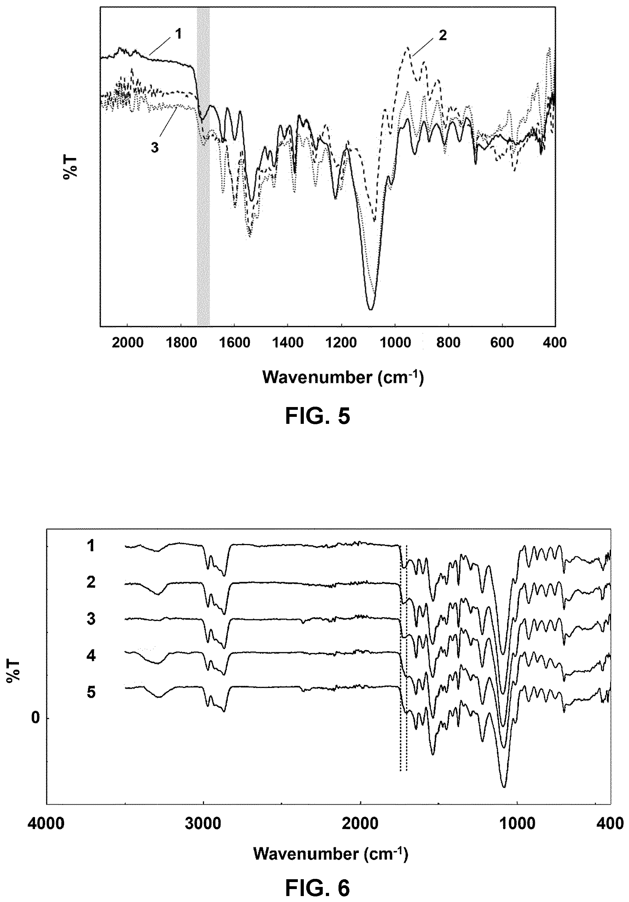

Figure 5 shows the ATR-FTIR spectra of: (1) ground polyurethane foam waste; (2) polyurethane foam synthesized from a commercial polyol mass for industrial use; and (3) polyurethane foam synthesized from a polyol mass recovered by means of a preferred embodiment of the method of the invention. The characteristic band of the urethane bond (1720-1740 cm⁻¹) is indicated by means of a grey strip.
Figure 6 shows the ATR-FTIR spectrum of a polyurethane foam mass (1) as well as those of the resulting solid fractions after applying the depolymerization process of the invention to said foam using a reaction medium without ionic liquid, or without a superbase, and with water as nucleophilic agent: 1,5-diazabicyclo [4.3.0] non-5-ene (DBN) at 15% w/w in water (2); tetramethylguanidine (TMG) at 15% w/w in water (3); [Bmim][CI] at 66% w/w in water (4); and [Bmim][Br] at 66% w/w in water (5). The characteristic band of the urethane bond (1700-1740 cm⁻¹) is represented delimited by two dashed vertical lines.

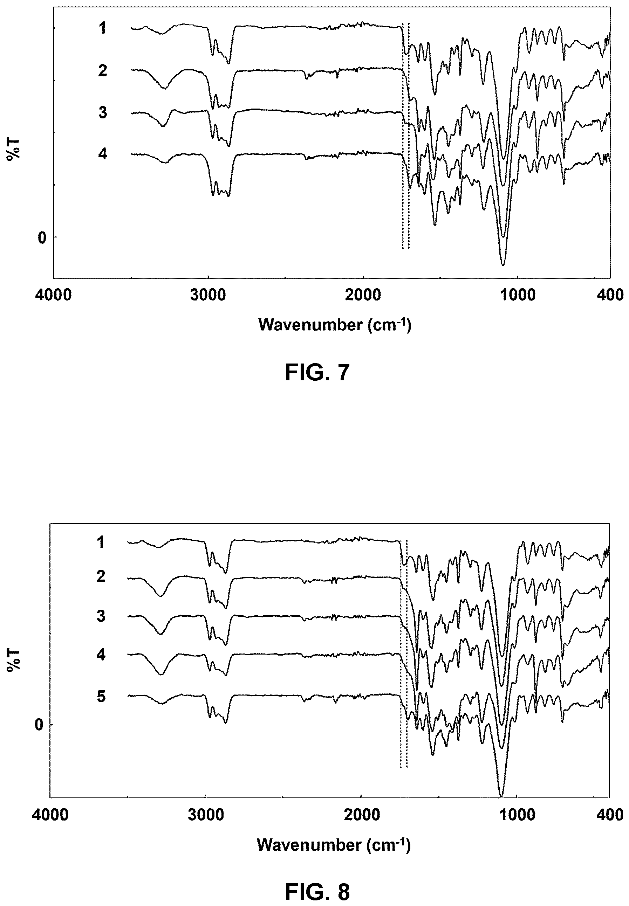

Figure 7 shows the ATR-FTIR spectrum of a polyurethane foam mass (1) as well as those of the resulting solid fractions recovered (2-4) after applying a depolymerization process to said foam according to a preferred embodiment of the present invention. Specifically, in a depolymerization reaction medium containing: 1-butyl-1-methylpyrrolidinium chloride ([BmPyrr][Cl]), DBU and water (2); 1-butyl-1-methylpiperidinium iodide ([BmPip][I]), DBU and water (3); or tetrabutylphosphonium bromide ([TBP][Br]), DBU and water (4), for 6 h at 95°C. The characteristic band of the urethane bond (1700-1740 cm⁻¹) is represented delimited by two dashed vertical lines.
Figure 8 shows the ATR-FTIR spectrum of a polyurethane foam mass (1) as well as those of the solid fractions recovered (2-5) after applying a depolymerization process to said foam according to a preferred embodiment of the present invention. Specifically, in a depolymerization reaction medium containing: [Bmim][Ac], TMG and water (2); [Bmim][CI], TMG and water (3); 1-allyl-3-methylimidazolium chloride ([Amim][Cl]), TMG and water (4); or [TBP][Br], TMG and water (5), for 6 h at 95°C. The characteristic band of the urethane bond (1700-1740 cm⁻¹) is represented delimited by two dashed vertical lines.

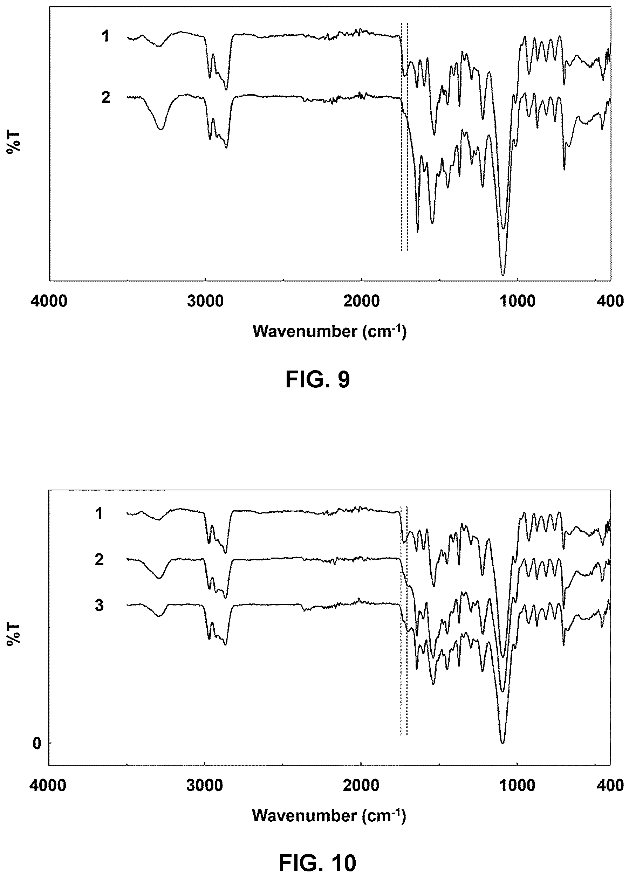

Figure 9 shows the ATR-FTIR spectra performed on a polyurethane foam mass (1) and on the solid polyol recovered after the depolymerization of said foam at 95°C for 6 h in a reaction medium containing [Bmim][Cl], DBN and water (2). The characteristic band of the urethane bond (1700-1740 cm⁻¹) is represented delimited by two dashed vertical lines.
Figure 10 shows the ATR-FTIR spectrum of a polyurethane foam mass (1) as well as those of the solid fractions recovered (2, 3) after applying a depolymerization process to said foam according to a preferred embodiment of the present invention. Specifically, in a depolymerization reaction medium containing: 1-butyl-2-methylpyridinium chloride ([BmPyr][Cl]), DBU and water (2); or N-ethylpyridinium bromide ([Epyr][Br]), DBU and water (3), for 6 h at 95°C. The characteristic band of the urethane bond (1700-1740 cm⁻¹) is represented delimited by two dashed vertical lines.

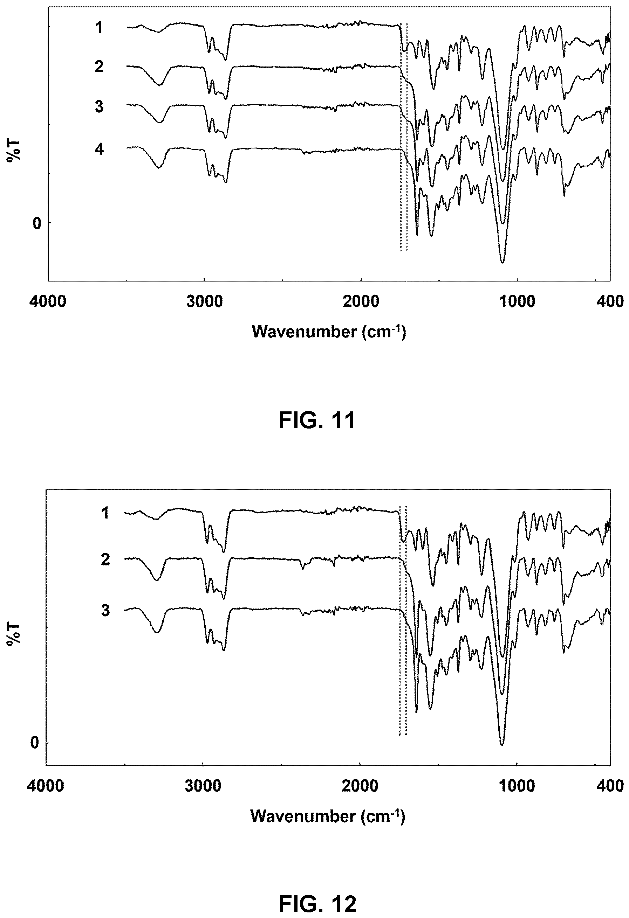

Figure 11 shows the ATR-FTIR spectrum of a polyurethane foam mass (1) as well as those of the solid fractions recovered (2-4) after applying a depolymerization process to said foam according to a preferred embodiment of the present invention. Specifically, in a depolymerization reaction medium containing: [Bmim][Ac], DBU and methanol (2); [Bmim][CI], TMG and ethanol (3); or [BmPip][I], TMG and ethanol (4), for 6 h at 95°C. The characteristic band of the urethane bond (1700-1740 cm⁻¹) is represented delimited by two dashed vertical lines.
Figure 12 shows the ATR-FTIR spectrum of a polyurethane foam mass (1) as well as those of the solid fractions recovered (2, 3) after applying a depolymerization process to said foam according to a preferred embodiment of the present invention. Specifically, in a depolymerization reaction medium containing: [Bmim][CI], DBU and 1-butylamine (2); or [TBP][Br], DBN and 1-butylamine (3). The characteristic band of the urethane bond (1700-1740 cm⁻¹) is represented delimited by two dashed vertical lines.

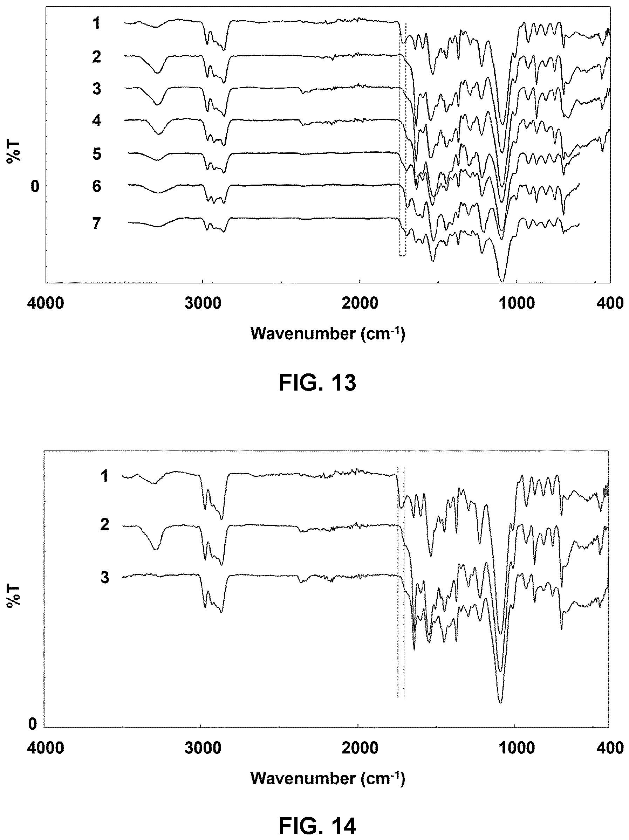

Figure 13 shows the ATR-FTIR spectrum of a polyurethane foam mass (1) as well as those of the solid fractions recovered (2-7) after applying a depolymerization process to said foam according to a preferred embodiment of the present invention. Specifically, in a depolymerization reaction medium containing: [Bmim][Cl], 1,5,7-triazabicyclo [4.4.0] dec-5-ene (TBD) and water (2); [BmPyrr][Cl], TBD and water (3); [BmPip][I], TBD and water (4); [BmPyr][Cl], TBD and water (5); [TBP][Br], TBD and water (6); or 1-ethyl-2-methylpyridinium bromide ([EmPyr][Br]), TBD and water (7). The characteristic band of the urethane bond (1700-1740 cm⁻¹) is represented delimited by two dashed vertical lines.
Figure 14 shows the ATR-FTIR spectrum of a polyurethane foam mass (1) as well as those of the solid fractions recovered (2, 3) after applying a depolymerization process to said foam according to a preferred embodiment of the present invention. Specifically, in a depolymerization reaction medium containing: [Bmim][CI], TBD and solketal (2); or [BmPyrr][Cl], TBD and solketal (3). The characteristic band of the urethane bond (1700-1740 cm⁻¹) is represented limited by two dashed vertical lines.

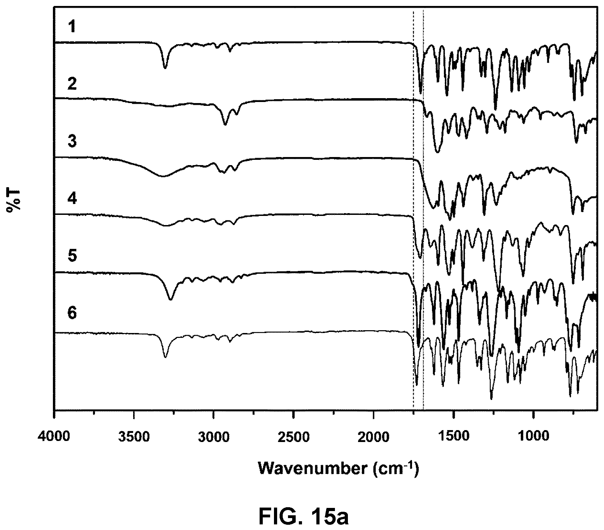

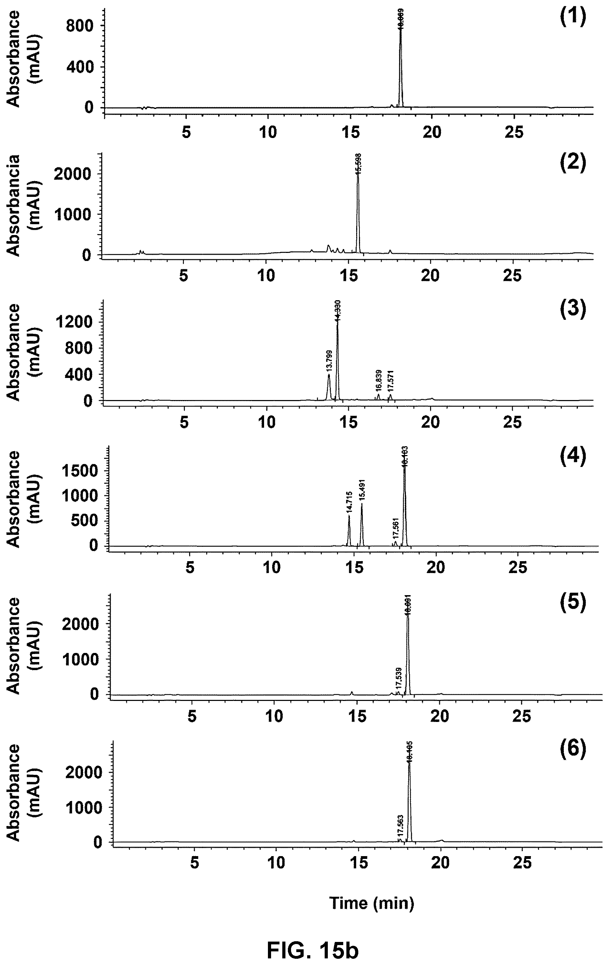

Figure 15a shows the ATR-FTIR spectrum of a mass of oxybis(ethane-2,1-diyl) bis(phenylcarbamate) as well as the products isolated after the rupture of the urethane bond upon subjecting said mass to 95°C for 4 h in a reaction medium containing: [Bmim][CI], DBU and water (2); [Bmim][CI], TBD and water (3); [Bmim][CI], pyrrolidine and water (4); [Bmim][CI], tert-butylamine and water (5); or [Bmim][CI], triethylamine and water (6). The characteristic band of the urethane bond (1700-1740 cm⁻¹) is represented delimited by two dashed vertical lines. Figure 15b shows the HPLC chromatograms of both said urethane mass (1) and the isolated products after the rupture of the urethane bond under the experimental conditions indicated above (2-6).

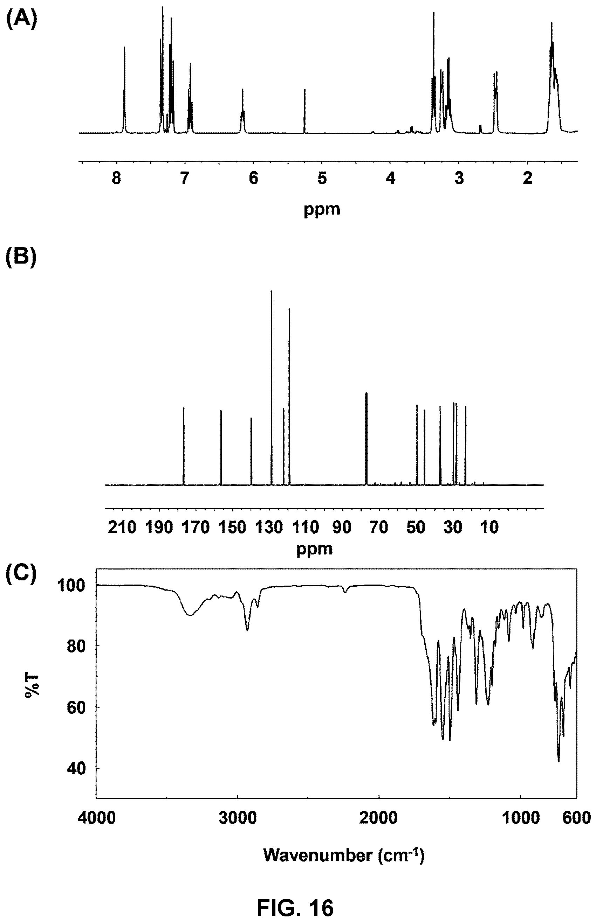

Figure 16 shows the spectra of: (A) ¹H-NMR, (B) ¹³C-NMR, and (C) ATR-FTIR of the product isolated after the rupture of the urethane bond of a mass of oxybis(ethane-2,1-diyl) bis(phenylcarbamate) upon subjecting same to 95°C for 4 h in a reaction medium containing [Bmim][CI] as ionic liquid, DBU as superbase, and water as nucleophilic agent.

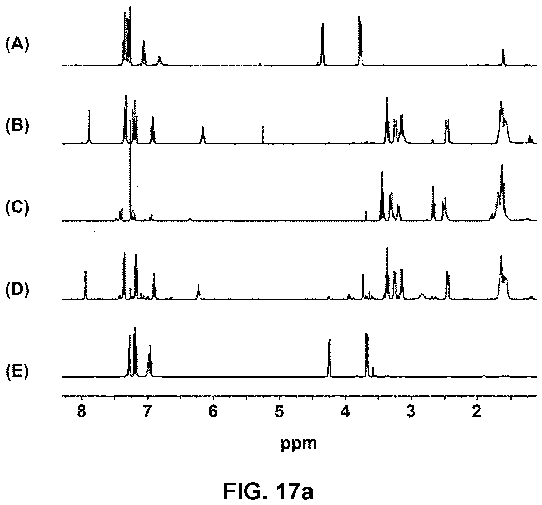

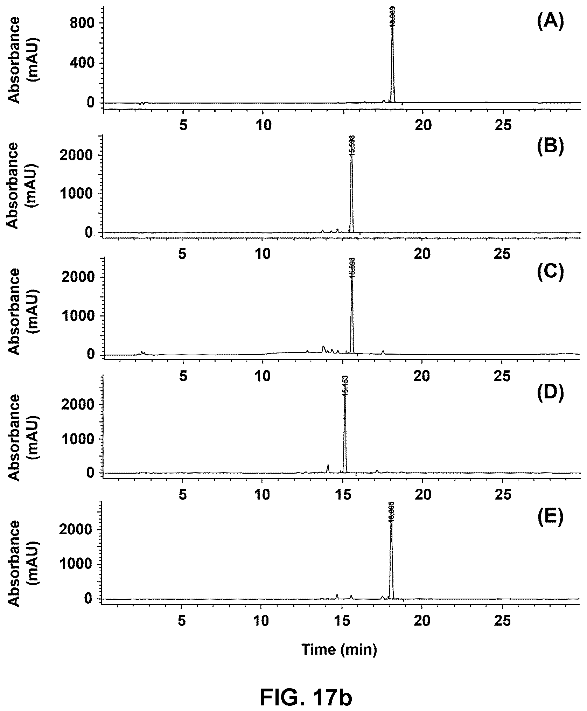

Figure 17a shows the ¹H-NMR spectra of: (A) oxybis(ethane-2,1-diyl) bis(phenylcarbamate); (B) product isolated after the rupture of the urethane bond of oxybis(ethane-2,1-diyl) bis(phenylcarbamate)); (C) reaction product obtained by using [Bmim][CI], water and 12.3 equivalents of DBU as reaction medium; (D) reaction product by using [Bmim][CI], water and 5 equivalents of DBU as reaction medium; (E) reaction product obtained by using [Bmim][CI] and water as reaction medium (in the absence of DBU). Figure 17b shows the HPLC chromatograms corresponding to the same compounds.

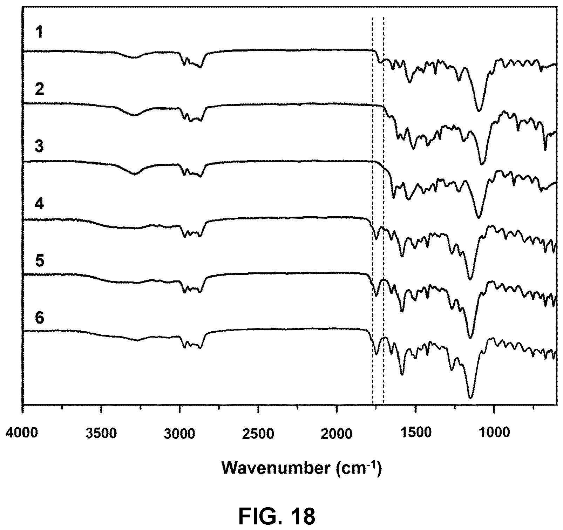

Figure 18 shows the ATR-FTIR spectrum of a polyurethane foam mass (1) as well as those of the solid fractions recovered (2-6) after applying a depolymerization process to said foam in a reaction medium containing: [Bmim][CI], DBU and water (2); [Bmim][CI], TBD and water (3); [Bmim][CI], pyrrolidine and water (4); [Bmim][Cl], tert-butylamine and water (5); or [Bmim][CI], triethylamine and water (6). The characteristic band of the urethane bond (1700-1740 cm⁻¹) is represented delimited by two dashed vertical lines.

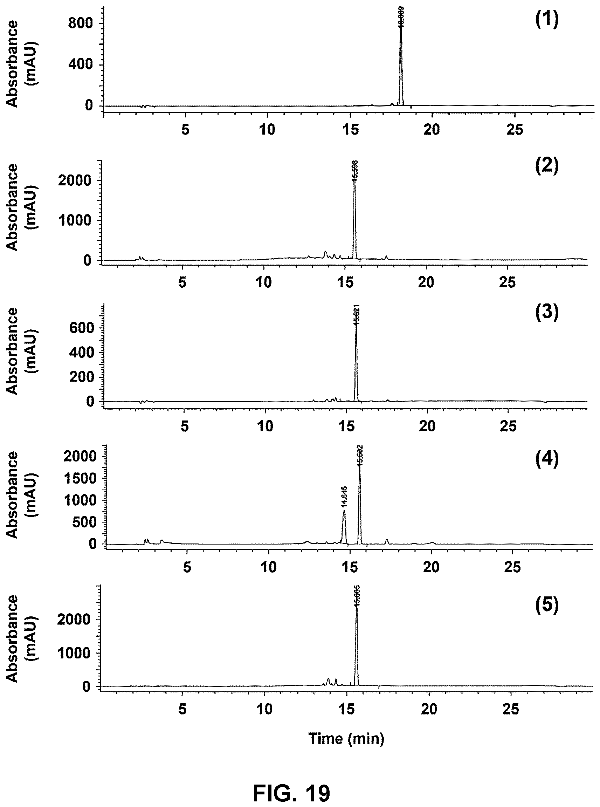

Figure 19 shows the HPLC chromatograms of both the mass of oxybis(ethane-2,1-diyl) bis(phenylcarbamate)) (1) and the products isolated after the rupture of the urethane bond upon subjecting said mass to 95°C for 4 h in a reaction medium containing: [Bmim][CI], DBU and water (2); [Bmim][Ac], DBU and water (3); [TBP][Br], DBU and water (4); or [BmPyr][Cl], DBU and water (5).

### DETAILED DESCRIPTION OF THE INVENTION

As described in previous sections, the object of the present invention relates to a method for depolymerizing a polyurethane mass by means of a hydrolysis reaction, an alcoholysis reaction, an aminolysis reaction, or any possible combination thereof, at temperatures less than 100°C and in a reaction medium comprising an ionic liquid, a basic catalyst, and a nucleophilic agent. Preferably, the polyurethane mass comprises a thermoset polyurethane and, more preferably, a polyurethane foam, generally from removed post-consumption products and post-production waste.

The ionic liquid comprises a cation with a high charge delocalization, preferably dialkylimidazolium, tetraalkylammonium, dialkylpiperidinium, alkylpyridinium, dialkylpyrrolidonium, and/or tetraalkylphosphonium, and/or an anion of a hydrophilic nature and without charge delocalization, which include, preferably, fluoride, chloride, bromide, iodide, acetate, trifluoroacetate, formate, and/or carbonate. More preferably, the ionic liquid used in the method of the invention comprises 1-butyl-3-methylimidazolium acetate, 1-ethyl-3-methylimidazolium acetate, 1-butyl-3-methylpyridinium chloride, 1-butyl-3-methylpyridinium bromide, 1-butyl-3-methylpyridinium iodide, 1-butyl-1-methylpiperidinium chloride, 1-butyl-1-methylpiperidinium bromide, 1-butyl-1-methylpiperidinium iodide, 1-butyl-3-methylimidazolium chloride, 1-butyl-3-methylimidazolium bromide, 1-butyl-3-methylimidazolium iodide, 1-ethyl-3-methylimidazolium chloride, 1-ethyl-3-methylimidazolium bromide, 1-ethyl-3-methylimidazolium iodide, 1-butyl-4-methylpyridinium chloride, 1-butyl-3-methylimidazolium formate, 1-hexyl-3-methylimidazolium chloride, 1-hexyl-3-methylimidazolium bromide, 1-hexyl-3-methylimidazolium iodide, 1-octyl-3-methylimidazolium chloride, 1-octyl-3-methylimidazolium bromide, 1-octyl-3-methylimidazolium iodide, 1-allyl-3-methylimidazolium chloride, 1-butyl-3-methyl-imidazolium acetate, 1-butyl-3-methylimidazolium bromide, 1-ethyl-2-methylpyridinium bromide, N-ethylpyridinium bromide, 1-butyl-3-methyl-imidazolium chloride, 1-butyl-1-methylpyrrolidinium chloride, 1-butyl-1-methylpiperidinium iodide, tetrabutylphosphonium bromide, tetrabutylphosphonium chloride, 1-butyl-2-methylpyridinium chloride, 1-ethyl-1-methyl-piperidinium chloride, or any possible combination thereof. Regarding its concentration in the reaction medium, the ionic liquid is in a proportion equal to or greater than 50% w/w with respect to the total weight of the depolymerization reaction medium; preferably between 50-85% w/w and, more preferably, between 65-80% w/w.

The basic catalyst comprises a superbase preferably containing at least a bicyclic amidine, a bicyclic amidine derivative, a guanidine, a guanidine derivative, or any possible combination thereof. These types of catalysts are more active in non-aqueous systems and/or with a low water content and have a higher stability over time compared to conventional basic catalysts, such as sodium hydroxide (NaOH) or potassium hydroxide (KOH), which are carbonated over time. Even more preferably, the basic catalyst used in the method of the invention comprises 1,8-diazabicyclo [5.4.0] undec-7-ene (DBU), 1,5-diazabicyclo [4.3.0] non-5-ene (DBN), 1,5,7-triazabicyclo [4.4.0] dec-5-ene (TBD), tetramethylguanidine (TMG), or any possible combination thereof.

The nucleophilic agent preferably comprises water (depolymerization reaction by hydrolysis), an alcohol (depolymerization reaction by alcoholysis), an amine (depolymerization reaction by aminolysis), or any possible combination thereof, in a proportion equal to or less than 15% w/w with respect to the total weight of the depolymerization reaction medium. More preferably, the nucleophilic agent comprises at least 80% water due to its higher nucleophilic nature, immiscibility with polyols, and ease of removal by evaporation. With respect to its concentration in the depolymerization reaction medium, the nucleophilic agent is preferably in a proportion between 1-15% w/w with respect to the weight of said medium, and, more preferably, between 5-15% w/w.

The mixture of the polyurethane mass and the reaction medium is incubated at a controlled temperature comprised between 50 and 100°C, preferably between 80-100°C, more preferably between 90 and 98°C, and, even more preferably, at 95°C, under constant stirring (mechanical, magnetic, or the like) for a time period comprised between 2 minutes and 10 hours; preferably between 2 and 8 hours, and, even more preferably, between 3 and 7 hours.

Next, the washing of said mixture is performed by adding a mass of a protic molecular solvent in an amount between 4 and 40 times more the weight of the reaction medium, preferably between 5 and 15 times. This washing of the mixture causes the precipitation of a polyol mass, a constituent of the polyurethane mass, due to its insolubility in said solvent. Preferably, the protic molecular solvent comprises water, methanol, ethanol, isopropanol, or any possible combination thereof, and, more preferably, at least 80% water.

The heterogenous mixture or suspension resulting after the addition of the protic molecular solvent is kept under constant stirring (mechanical, magnetic, or the like) for at least 1 hour at a temperature comprised between 20 and 60°C, being recommended a time period of 2 h at 40°C. Then, the reaction mixture is subjected to a separation process, typically filtration or centrifugation, allowing two fractions to be obtained: a first fraction or semisolid fraction, and a second fraction or single-phase liquid fraction.

The single-phase liquid fraction contains all the elements of the reaction medium soluble in the protic molecular solvent, namely, the ionic liquid and the catalyst. Said mixture can be filtered through a hydrophobic adsorbent, such as activated carbon or polystyrene resin, to remove any diamine residue and prevent the darkening of the fraction. After the filtration process, said fraction can be concentrated, for example, by means of evaporation at reduced pressure to remove the water present to a final concentration of less than 1% w/w, so that it can be reused directly in a new process for chemical polyurethane depolymerization.

The semisolid fraction mainly contains a polyol mass. To check its molecular nature, the fraction is subjected to a drying process, preferably by lyophilization or evaporation at reduced pressure, which allows reducing the water content to a level lower than 1% w/w. Subsequently, three different analytical tests can be performed to verify the complete depolymerization of the polyurethane mass; namely, solubility in organic solvents, attenuated total reflectance combined with Fourier transform infrared spectroscopy (ATR-FTIR), and hydroxyl value determination.

Polyurethane is insoluble in all the conventional polar organic solvents, such as acetone or dimethyl sulfoxide (DMSO), unlike polyols. This difference allows checking the nature of the semisolid fraction resulting after the depolymerization process of the invention by means of a simple solubility test. Preferably, 20 mg of the resulting semisolid fraction are filtered and dried, and then resuspended in 2 mL of DMSO. If depolymerization is complete, the semisolid fraction will dissolve completely in the organic solvent, demonstrating the loss of polymeric integrity.

The molecular confirmation of the success of the depolymerization reaction can be performed by ATR-FTIR analysis of the first dry fraction. The absence of the characteristic band of the carbonyl group (CO) forming the urethane bond (-NH-CO-O-) between 1700 and 1740 cm⁻¹ would demonstrate the correct depolymerization of the polyurethane mass.

Likewise, determining the hydroxyl value (mg KOH/g of sample) by titration of the first dry fraction, with phthalic anhydride/pyridine according to the ASTM D-4274-16 (2016) standard, allows quantifying the presence of hydroxyl groups in said fraction. Since polyols contain multiple hydroxyl groups, the higher said value is, the greater the amount of polyols present in the fraction, demonstrating the correct depolymerization of the polyurethane mass according to the method of the invention.

### Examples

Various embodiments of the invention are detailed below, which demonstrate that, to carry out the depolymerization of a polyurethane mass at temperatures between 50-100°C for a time period less than 10 h (so that the recovered polyol does not require a later step of purification), it is strictly necessary that:
- the polymerization reaction medium comprises an ionic liquid, a superbase and a nucleophilic agent;
- the ionic liquid is the major component of said reaction medium;
- the basic catalyst is a superbase;
- the concentration of nucleophilic agent is equal to or less than 15% w/w with respect to the total weight of the depolymerization reaction medium;
- a protic molecular solvent is used in an amount between 4 and 40 times more the weight of the depolymerization reaction medium to stop the reaction.

To that end, in addition to visual examples (see Examples 1-3), the results of the analytical tests described above will be shown to verify the complete depolymerization of a polyurethane mass (that is, solubility test, ATR-FTIR spectroscopy analysis, and determination of the hydroxyl value) when using depolymerization reaction media:
- with the same superbase and nucleophilic agent, but different ionic liquid (see Examples 4, 7 and 10);
- with the same ionic liquid and nucleophilic agent, but different superbase (see Examples 4, 8, 9, and 14);
- with the same ionic liquid and superbase, but different nucleophilic agent (see Examples 4, 8, 11, 12, 13, 14, and 15);
- which are incomplete, either without an ionic liquid or without a superbase (see Example 6);
- with a base as a catalyst instead of a superbase (see Example 20).

Likewise, it is demonstrated that the polyol fraction recovered by means of the method of the invention can be reused as starting material for the synthesis of a new polyurethane by means of any protocol known in the state of the art (see Example 5).

Finally, the effect of the components of the reaction medium was also evaluated in a simple urethane model: oxybis(ethane-2,1-diyl) bis(phenylcarbamate), which was synthesized and purified according to the method described in Motokucho, S. et al. J. Polym. Sci. A Polym. Chem. 2017, 55 (12), p. 2004-2010. The use of this model instead of a polyurethane foam as the starting material allows monitoring the evolution of the transformation of the carbamate groups by means of Nuclear Magnetic Resonance (NMR) or High-Performance Liquid Chromatography (HPLC) techniques, providing evidence of the rupture of said bonds that are key for the depolymerization of polyurethane.

Tables 1 and 2 include the abbreviations for the ionic liquids and superbases used in the depolymerization reaction medium of the different embodiments of the invention shown herein.

**Table 1. Ionic liquids used in the embodiments of the invention.**

| **Abbrev.** | **Name of the compound** |
|---|---|
| [Amim][Cl] | 1-allyl-3-methylimidazolium chloride |
| [Bmim][Ac] | 1-butyl-3-methyl-imidazolium acetate |
| [Bmim][Br] | 1-butyl-3-methyl-imidazolium bromide |
| [Bmim][Cl] | 1-butyl-3-methyl-imidazolium chloride |
| [BmPip][I] | 1-butyl-1-methylpiperidinium iodide |
| [BmPyr][Cl] | 1-butyl-2-methylpyridinium chloride |
| [BmPyrr][Cl] | 1-butyl-1-methylpyrrolidinium chloride |
| [Emim][Cl] | 1-ethyl-3-methylimidazolium chloride |
| [EmPyr][Br] | 1-ethyl-2-methylpyridinium bromide |
| [EPyr][Br] | N-ethylpyridinium bromide |
| [Omim][Cl] | 1-octyl-3-methylimidazolium chloride |
| [TBP][Br] | Tetrabutylphosphonium bromide |

**Table 2. Superbases used in the embodiments of the invention.**

| **Abbrev.** | **Name of the compound** |
|---|---|
| DBN | 1,5-diazabicyclo [4.3.0] non-5-ene |
| DBU | 1,8-diazabicyclo [5.4.0] undec-7-ene |
| TBD | 1,5,7-triazabicyclo [4.4.0] dec-5-ene |
| TMG | N,N,N',N'- tetramethylguanidine |

### Example 1: Depolymerization of a polyurethane foam mass (PU) at 95°C for 4 h in a depolymerization reaction medium containing [Bmim][Cl], [Bmim][Br] [Bmim][I] [Emim][Cl], or [Omim][Cl] as ionic liquid, DBU as superbase and water as nucleophilic agent.

By way of illustration of the present invention, Figure 1 shows the evolution of five different reaction media throughout the depolymerization process of a polyurethane foam according to preferred embodiments of the method of the invention. Specifically, depolymerization reaction media based on the combination of [Bmim][Cl], [Bmim][Br] [Bmim][I] [Emim][Cl], or [Omim][Cl] (65% w/w, ionic liquid) with DBU (20% w/w, superbase) and water (15% w/w, nucleophilic agent) have been used to treat a ground polyurethane foam mass (25% w/w, with respect to the total mass of the depolymerization reaction medium). The images show the appearance of the depolymerization reaction mixtures at the start of the reaction (Figure 1 A), and after 4 hours of incubation with magnetic stirring at 95°C (Figure 1B).

### Example 2: Evolution over time of the depolymerization of a PU foam mass in a depolymerization reaction medium containing [Bmim][Cl] as ionic liquid, DBU as superbase and water as nucleophilic agent.

Figure 2 shows the evolution over time of a polyurethane foam mass (0.2 g) immersed in 3 g of depolymerization reaction medium, based on [Bmim][CI] (65% w/w), DBU (20% w/w), and water (15% w/w), under stirring at 95°C by means of images taken over 2 min.

### Example 3: Separation of the polyol recovered after the depolymerization of a PU foam mass at 95°C for 4 h in a depolymerization reaction medium containing [Bmim][Cl] or [Bmim][Br] as ionic liquid, DBU as superbase and water as nucleophilic agent.

Figure 3 shows the separation of the polyol recovered by centrifugation after the depolymerization of a ground polyurethane foam mass (25% w/w with respect to the total mass of the system) at 95°C for 4 h in a depolymerization reaction medium containing [Bmim][CI], or [Bmim][Br] (65% w/w), DBU (20% w/w) and water (15% w/w) under constant stirring. After that time elapsed, 20 mL of water were added.

### Example 4: Depolymerization of a PU foam mass at 95°C for 4 h in a depolymerization reaction medium containing [Bmim][Ac], [Bmim][Cl] or [Emim][Cl] as ionic liquid, DBU as superbase and water as nucleophilic agent.

In three 50 mL screw cap containers, 1 g of [Bmim][Ac], [Bmim][CI], or [Emim][Cl] was added, respectively, and mixed with 0.3 g of DBU, 0.25 g of water, and 0.4 g of ground polyurethane foam in each of them. The three containers were kept closed and under mechanical stirring for 4 h at 95°C. After that period has elapsed, 20 mL of water were added to each of the containers, keeping them under stirring for 1 h at room temperature. Then, each of the resulting suspensions was centrifuged at 7000 rpm for 20 min. The resulting precipitates were separated by means of decantation and filtration through nylon membranes (0.45 microns), and finally dried in a vacuum oven at 80°C to constant weight. The dry products were analyzed by means of the following control tests:
▪ **Level Control 1 *- Solubility test.*** The addition of 20 mg of each of the dry polyols obtained over 2 mL of dimethyl sulfoxide (DMSO) allowed the complete solubilization thereof, resulting in translucent solutions in all cases. Since polyurethane is not soluble in DMSO, and polyol is, this result indicates that the depolymerization of the polyurethane foam mass has taken place.
▪ **Level Control 2 *- ATR-FTIR spectroscopy analysis*** (see Figure 4). Each of the dry polyols obtained was analyzed by ATR-FTIR, comparing their spectra (3-5) with that of the original polyurethane foam mass (1) and that of a commercial polyol mass (2). The characteristic band of the urethane bond (1700-1740 cm⁻¹) was not detected in any of the cases (see spectra 3, 4 and 5), obtaining a signal similar to that of the commercial polyol (spectrum 2). This confirms the correct depolymerization of the polyurethane foam mass according to the method of the invention.
▪ **Level Control 3 *-Hydroxyl value determination.*** By titration of the different dry polyols with phthalic anhydride/pyridine, based on the ASTM D-4274-16 (2016) standard, , the following hydroxyl values were obtained:
   - PU standard: 9.45 mg KOH / g polyol
   - Reaction medium [Bmim][Ac] + DBU + water: 57.41 mg KOH / g polyol
   - Reaction medium [Bmim][CI] + DBU + water: 52.52 mg KOH / g polyol
   - Reaction medium [Emim][Cl] + DBU + water: 55.61 mg KOH / g polyol
   A higher hydroxyl value represents a higher concentration of polyols in the sample, since said compounds are characterized by having multiple hydroxyl groups. Therefore, since the hydroxyl values obtained for the different solid fractions are much higher than that of the starting polyurethane foam mass, the correct depolymerization thereof according to these preferred embodiments of the method of the invention is once again confirmed.

### Example 5: Reuse of the polyol fraction recovered by means of the method of the invention in the synthesis of a new polyurethane.

Figure 4 shows the ATR-FTIR spectra of: (1) ground polyurethane foam waste; (2) polyurethane foam synthesized from a commercial polyol mass for industrial use; and (3) polyurethane foam synthesized from a polyol mass recovered by means of a preferred embodiment of the method of the invention. Namely, for said polyol mass recovered according to a preferred embodiment of the method of the invention, a depolymerization reaction medium that comprised 65% w/w of [Bmim][Cl], 20% w/w of DBU and 15% w/w of water was used. The depolymerization reaction was performed by combining 40% of polyurethane foam waste and 60% of the depolymerization reaction medium, being kept under constant stirring for 4 hours at 95°C. As can be observed, the three polymeric products have a band in the characteristic region of the urethane bond (1700-1740 cm⁻¹), which demonstrates that the first fraction obtained by means of the method of the invention can be used as the starting material for the synthesis of a new polyurethane by means of any protocol known in the state of the art.

### Example 6: Depolymerization of a PU foam mass in incomplete depolymerization reaction media.

In four 50 mL screw cap containers, four incomplete depolymerization reaction media were prepared by adding 0.4 g of DBN, or 0.4 g of TMG, or 2.4 g of [Bmim][CI], or 2.4 g of [Bmim][Br], respectively, that were mixed with 2.6 g, 2.6 g, 0.6 g, or 0.6 g of water, respectively. Finally, 1 g of ground polyurethane foam was added in each of them. The four containers were kept closed and under mechanical stirring for 6 h at 95°C, and then 20 mL of water were added to stop the reaction. The precipitated products were subjected to the same method of sample separation, drying and analysis described in Example 4, showing the following results:
▪ **Level Control 1 *- Solubility test.*** The addition of 20 mg of each of the dry solids obtained over 2 mL of DMSO resulted in an insoluble precipitate in all cases, which indicates that the depolymerization of the polyurethane foam mass could not be carried out under these conditions.
▪ **Level Control 2 *- ATR-FTIR spectroscopy analysis*** (see Figure 6). Each of the dry products obtained was analyzed by ATR-FTIR, comparing their spectra (2-5) with that of the original polyurethane foam mass (1). The characteristic band of the urethane bond (1700-1740 cm⁻¹) was detected in all cases (spectra 2-5), which allows identifying them as non-depolymerized polyurethanes. This experiment demonstrates the inefficiency of the process at temperatures less than 100°C when either the ionic liquid or the superbase is not present simultaneously in the depolymerization reaction medium.
▪ **Level Control 3 *-Hydroxyl value determination.*** Since the hydroxyl values obtained for the different solid fractions are very similar to that of the starting polyurethane foam mass, the inefficiency of the process for depolymerization is again confirmed under these conditions:
   - PU standard: 9.45 mg KOH / g polyol
   - Reaction medium [Bmim][CI] + water: 9.52 mg KOH / g polyol
   - Reaction medium [Bmim][Br] + water: 9.85 mg KOH / g polyol
   - Reaction medium DBU + water: 11.25 mg KOH / g polyol
   - Reaction medium DBN + water: 12.75 mg KOH / g polyol

### Example 7: Depolymerization of PU foam at 95°C for 6 h in a depolymerization reaction medium containing [BmPyrr][Cl], [BmPip][I] or [TBP][Br] as ionic liquid, DBU as superbase and water as nucleophilic agent.

In three 50 mL screw cap containers, 2 g of [BmPyrr][Cl], [BmPip][I] or [TBP][Br] were added, respectively, and mixed with 0.4 g of DBU, 0.6 g of water, and 1 g of ground polyurethane foam, in each of them. The three containers were kept closed and under mechanical stirring for 6 h at 95°C, and then 20 mL of water were added to stop the reaction. The precipitated products were subjected to the same method of sample separation, drying and analysis described in Example 4, showing the following results:
▪ **Level Control 1 *- Solubility test.*** The addition of 20 mg of each of the dry polyols obtained over 2 mL of DMSO allowed the complete solubilization thereof, resulting in translucent solutions in all cases. This indicates that the depolymerization of the polyurethane foam mass has taken place.
▪ **Level Control 2 *- ATR-FTIR spectroscopy analysis*** (see Figure 7). Each of the dry polyols obtained was analyzed by ATR-FTIR, comparing their spectra (2-4) with that of the original polyurethane foam mass (1). The characteristic band of the urethane bond (1700-1740 cm⁻¹) was not detected in any of the cases: reaction medium [BmPyrr][Cl] + DBU + water (spectrum 2); reaction medium [BmPip][I] + DBU + water (spectrum 3); reaction medium [TBP][Br] + DBU + water (spectrum 4). This confirms the correct depolymerization of the polyurethane foam mass according to the method of the invention.
▪ **Level Control 3 *- Hydroxyl value determination.*** Since the hydroxyl values obtained for the different solid fractions are much higher than that of the starting polyurethane foam mass, the correct depolymerization thereof according to these preferred embodiments of the method of the invention is again confirmed:
   - PU standard: 9.45 mg KOH / g polyol
   - Reaction medium [BmPyrr][Cl] + DBU + water: 97.04 mg KOH / g polyol
   - Reaction medium [BmPip][I] + DBU + water: 78.2 mg KOH / g polyol
   - Reaction medium [TBP][Br] + DBU + water: 58.71 mg KOH / g polyol

### Example 8: Depolymerization of a PU foam mass at 95°C for 6 h in a depolymerization reaction medium containing [Bmim][Ac], [Bmim][Cl], [Amim][Cl], or [TBP][Br] as ionic liquid, TMG as superbase and water as nucleophilic agent.

In four 50 mL screw cap containers, 2 g of [Bmim][Ac], [Bmim][CI], [Amim][Cl], or [TBP][Br] were added, respectively, and mixed with 0.4 g of TMG, 0.6 g of water, and 1 g of shredded polyurethane foam, in each of them. The four containers were kept closed and under mechanical stirring for 6 h at 95°C, and then 20 mL of water were added to stop the reaction. The precipitated products were subjected to the same method of sample separation, drying and analysis described in Example 4, showing the following results:
▪ **Level Control 1 *- Solubility test.*** The addition of 20 mg of each of the dry polyols obtained over 2 mL of DMSO allowed the complete solubilization thereof, resulting in translucent solutions in all cases. This indicates that the depolymerization of the polyurethane foam mass has taken place.
▪ **Level Control 2 *- ATR-FTIR spectroscopy analysis*** (see Figure 8). Each of the dry polyols obtained was analyzed by ATR-FTIR, comparing their spectra (2-5) with that of the original polyurethane foam mass (1). The characteristic band of the urethane bond (1700-1740 cm⁻¹) was not detected in any of the cases: reaction medium [Bmim][Ac] + TMG + water (spectrum 2); reaction medium [Bmim][CI] + TMG + water (spectrum 3); reaction medium [Amim][Cl] + TMG + water (spectrum 4); reaction medium [TBP][Br] + TMG + water (spectrum 5). This confirms the correct depolymerization of the polyurethane foam mass according to the method of the invention.
▪ **Level Control 3 *- Hydroxyl value determination.*** Since the hydroxyl values obtained for the different solid fractions are much higher than that of the starting polyurethane foam mass, the correct depolymerization thereof according to these preferred embodiments of the method of the invention is again confirmed:
   - PU standard: 9.45 mg KOH / g polyol
   - Reaction medium [Bmim][Ac] + TMG + water: 66.83 mg KOH / g polyol
   - Reaction medium [Bmim][CI] + TMG + water: 57.75 mg KOH / g polyol
   - Reaction medium [Amim][Cl] + TMG + water: 67.43 mg KOH / g polyol
   - Reaction medium [TBP][Br] + TMG + water: 58.56 mg KOH / g polyol

### Example 9: Depolymerization of a PU foam mass at 95°C for 6 h in a depolymerization reaction medium containing [Bmim][Cl] as ionic liquid, DBN as superbase and water as nucleophilic agent.

2 g of [Bmim][CI] were added and mixed with 0.4 g of DBN, 0.6 g of water, and 1 g of shredded polyurethane foam in a 50 mL screw cap container. The container was kept closed and under mechanical stirring for 6 h at 95°C, and then 20 mL of water were added to stop the reaction. The precipitated product was subjected to the same method of sample separation, drying and analysis described in Example 4, showing the following results:
▪ **Level Control 1 *- Solubility test.*** The addition of 20 mg of the dry polyol obtained over 2 mL of DMSO allowed the complete solubilization thereof, resulting in a translucent solution. This indicates that the depolymerization of the polyurethane foam mass has taken place.
▪ **Level Control 2 *- ATR-FTIR spectroscopy analysis*** (see Figure 9). The dry polyol obtained was analyzed by ATR-FTIR, comparing its spectrum (2) with that of the original polyurethane foam mass (1). The characteristic band of the urethane bond (1700-1740 cm⁻¹) was not detected, confirming the correct depolymerization of the polyurethane foam mass according to the method of the invention.
▪ **Level Control 3 *- Hydroxyl value determination.*** Since the hydroxyl value obtained for the solid fraction is much higher than that of the starting polyurethane foam mass, the correct depolymerization thereof according to this preferred embodiment of the method of the invention is again confirmed:
   - PU standard: 9.45 mg KOH / g polyol
   - Reaction medium [Bmim][CI] + DBN + water: 81.51 mg KOH / g polyol

### Example 10: Depolymerization of a PU foam mass at 95°C for 6 h in a depolymerization reaction medium containing [BmPyr][Cl] or [EPyr][Br] as ionic liquid, DBU as superbase and water as nucleophilic agent.

In two 50 mL screw cap containers, 2 g of [BmPyr][Cl] or [Epyr][Br] were added, respectively, and mixed with 0.4 g of DBU, 0.6 g of water, and 1 g of ground polyurethane foam, in each of them. The two containers were kept closed and under mechanical stirring for 6 h at 95°C, and then 20 mL of water were added to stop the reaction. The precipitated products were subjected to the same method of sample separation, drying and analysis described in Example 4, showing the following results:
▪ **Level Control 1 *- Solubility test.*** The addition of 20 mg of each of the dry polyols obtained over 2 mL of DMSO allowed the complete solubilization thereof, resulting in translucent solutions in all cases. This indicates that the depolymerization of the polyurethane foam mass has taken place.
▪ **Level Control 2 *- ATR-FTIR spectroscopy analysis*** (see Figure 10). Each of the dry polyols obtained was analyzed by ATR-FTIR, comparing their spectra (2, 3) with that of the original polyurethane foam mass (1). The characteristic band of the urethane bond (1700-1740 cm⁻¹) was not detected in any of the cases: reaction medium [BmPyr][Cl] + DBU + water (spectrum 2); reaction medium [Epyr][Br] + DBU + water (spectrum 3). This confirms the correct depolymerization of the polyurethane foam mass according to the method of the invention.
▪ **Level Control 3 *- Hydroxyl value determination.*** Since the hydroxyl values obtained for the different solid fractions are much higher than that of the starting polyurethane foam mass, the correct depolymerization thereof according to these preferred embodiments of the method of the invention is again confirmed:
   - PU standard: 9.45 mg KOH / g polyol
   - Reaction medium [BmPyr][Cl] + DBU + water: 66.28 mg KOH / g polyol
   - Reaction medium [Epyr][Br] + DBU + water: 67.36 mg KOH / g polyol

### Example 11: Depolymerization of a PU foam mass at 95°C for 6 h in a depolymerization reaction medium containing [Bmim][Ac] as ionic liquid, DBU as superbase and methanol as nucleophilic agent.

2 g of [Bmim][Ac] were added and mixed with 0.4 of DBU, 0.6 g of methanol, and 1 g of ground polyurethane foam in a 50 mL screw cap container. The container was kept closed and under mechanical stirring for 6 h at 95°C, and then 20 mL of water were added to stop the reaction. The precipitated product was subjected to the same method of sample separation, drying and analysis described in Example 4, showing the following results:
▪ **Level Control 1 *- Solubility test.*** The addition of 20 mg of the dry polyol obtained over 2 mL of DMSO allowed the complete solubilization thereof, resulting in a translucent solution. This indicates that the depolymerization of the polyurethane foam mass has taken place.
▪ **Level Control 2 *- ATR-FTIR spectroscopy analysis*** (see Figure 11). The dry polyol obtained was analyzed by ATR-FTIR, comparing its spectrum (2) with that of the original polyurethane foam mass (1). The characteristic band of the urethane bond (1700-1740 cm⁻¹) was not detected, confirming the correct depolymerization of the polyurethane foam mass according to the method of the invention.
▪ **Level Control 3 *- Hydroxyl value determination.*** Since the hydroxyl value obtained for the solid fraction is much higher than that of the starting polyurethane foam mass, the correct depolymerization thereof according to this preferred embodiment of the method of the invention is again confirmed:
   - PU standard: 9.45 mg KOH / g polyol
   - Reaction medium [Bmim][Ac] + DBU + methanol: 62.06 mg KOH / g polyol

### Example 12: Depolymerization of a PU foam mass at 95°C for 6 h in a depolymerization reaction medium containing [Bmim][Cl] or [BmPip][I] as ionic liquid, TMG as superbase and ethanol as nucleophilic agent.

In two 50 mL screw cap containers, 2 g of [Bmim][CI] or [BmPip][I] were added, respectively, and mixed with 0.4 g of TMG, 0.6 g of ethanol, and 1 g of ground polyurethane foam. The two containers were kept closed and under mechanical stirring for 6 h at 95°C, and then 20 mL of water were added to each of them to stop the reaction. The precipitated products were subjected to the same method of sample separation, drying and analysis described in Example 4, showing the following results:
▪ **Level Control 1 *- Solubility test.*** The addition of 20 mg of each of the dry polyols obtained over 2 mL of DMSO allowed the complete solubilization thereof, resulting in translucent solutions in all cases. This indicates that the depolymerization of the polyurethane foam mass has taken place.
▪ **Level Control 2 *- ATR-FTIR spectroscopy analysis*** (see Figure 11). Each of the dry polyols obtained was analyzed by ATR-FTIR, comparing their spectra (3, 4) with that of the original polyurethane foam mass (1). The characteristic band of the urethane bond (1700-1740 cm⁻¹) was not detected in any of the cases: reaction medium [Bmim][CI] + TMG + ethanol (spectrum 3); reaction medium [BmPip][I] + TMG + ethanol (spectrum 4). This confirms the correct depolymerization of the polyurethane foam mass according to the method of the invention.
▪ **Level Control 3 *- Hydroxyl value determination.*** Since the hydroxyl values obtained for the different solid fractions are much higher than that of the starting polyurethane foam mass, the correct depolymerization thereof according to these preferred embodiments of the method of the invention is again confirmed:
   - PU standard: 9.45 mg KOH / g polyol
   - Reaction medium [Bmim][CI] + TMG + ethanol: 61.23 mg KOH / g polyol
   - Reaction medium [BmPip][I] + TMG + ethanol: 54.92 mg KOH / g polyol

### Example 13: Depolymerization of a PU foam mass at 95°C for 6 h in a depolymerization reaction medium containing 1-butylamine as nucleophilic agent, [Bmim][Cl] or [TBP][Br] as ionic liquid, and DBU or DBN as superbase.

2 g of [Bmim][CI] were added and mixed with 0.4 g of DBU in a 50 mL screw cap container. In a similar container, 2 g of [TBP][Br] and 0.4 g of DBN were added. Finally, 0.6 g of 1-butylamine and 1 g of ground polyurethane foam were added to each of the containers, being kept closed and under mechanical stirring for 6 h at 95°C. Then, 20 mL of water were added to each container to stop the depolymerization reactions. The precipitated products were subjected to the same method of sample separation, drying and analysis described in Example 4, showing the following results:
▪ **Level Control 1 *- Solubility test.*** The addition of 20 mg of each of the dry polyols obtained over 2 mL of DMSO allowed the complete solubilization thereof, resulting in translucent solutions in all cases. This indicates that the depolymerization of the polyurethane foam mass has taken place.
▪ **Level Control 2 *- ATR-FTIR spectroscopy analysis*** (see Figure 12). Each of the dry polyols obtained was analyzed by ATR-FTIR, comparing their spectra (2, 3) with that of the original polyurethane foam mass (1). The characteristic band of the urethane bond (1700-1740 cm⁻¹) was not detected in any of the cases: reaction medium [Bmim][CI] + DBU + 1-butylamine (spectrum 2); reaction medium [TBP][Br] + DBN + 1-butylamine (spectrum 3). This confirms the correct depolymerization of the polyurethane according to the method of the invention.
▪ **Level Control 3 *- Hydroxyl value determination.*** Since the hydroxyl values obtained for the different solid fractions are much higher than that of the starting polyurethane foam mass, the correct depolymerization thereof according to these preferred embodiments of the method of the invention is again confirmed:
   - PU standard: 9.45 mg KOH / g polyol
   - Reaction medium [Bmim][CI] + DBU + 1-butylamine: 90.04 mg KOH / g polyol
   - Reaction medium [TBP][Br] + DBN + 1-butylamine: 85.56 mg KOH / g polyol

### Example 14: Depolymerization of a PU foam mass at 95°C for 6 h in a depolymerization reaction medium containing [Bmim][Cl], [BmPyrr][Cl], [BmPip][I], [BmPyr][Cl], [TBP][Br], or [EmPyr][Br] as ionic liquid, TBD as superbase, and water as nucleophilic agent.

In six 50 mL screw cap, 2 g of [Bmim][CI], [BmPyrr][Cl], [BmPip][I], [BmPyr][Cl], [TBP][Br], or [EmPyr][Br] were added, respectively. Next, 0.4 g of TBD, 0.6 g of water, and 1 g of ground polyurethane foam were added in each of them, being kept closed and under mechanical stirring for 6 h at 95°C. Then, 20 mL of water were added to each container to stop the reaction. The precipitated products were subjected to the same method of sample separation, drying and analysis described in Example 4, showing the following results:
▪ **Level Control 1 *- Solubility test.*** The addition of 20 mg of each of the dry polyols obtained over 2 mL of DMSO allowed the complete solubilization thereof, resulting in translucent solutions in all cases.
▪ **Level Control 2 *- ATR-FTIR spectroscopy analysis*** (see Figure 13). Each of the dry polyols obtained was analyzed by ATR-FTIR, comparing their spectra (2-7) with that of the original polyurethane foam mass (1). The characteristic band of the urethane bond (1700-1740 cm⁻¹) was not detected in any of the cases: reaction medium [Bmim][CI] + TBD + water (spectrum 2); reaction medium [BmPyrr][Cl] + TBD + water (spectrum 3); reaction medium [BmPip][I] + TBD + water (spectrum 4); reaction medium [BmPyr][Cl] + TBD + water (spectrum 5); reaction medium [TBP][Br] + TBD + water (spectrum 6); reaction medium [EmPyr][Br] + TBD + water (spectrum 7). This confirms the correct depolymerization of the polyurethane foam mass according to the method of the invention.
▪ **Level Control 3 *- Hydroxyl value determination.*** Since the hydroxyl values obtained for the different solid fractions are much higher than that of the starting polyurethane foam mass, the correct depolymerization thereof according to these preferred embodiments of the method of the invention is again confirmed:
   - PU standard: 9.45 mg KOH / g polyol
   - Reaction medium [Bmim][CI] + TBD + water: 96.61 mg KOH / g polyol
   - Reaction medium [BmPyrr][Cl] + TBD + water: 79.66 mg KOH / g polyol
   - Reaction medium [BmPip][I] + TBD + water: 111.46 mg KOH / g polyol
   - Reaction medium [BmPyr][Cl] + TBD + water: 66.51 mg KOH / g polyol
   - Reaction medium [TBP][Br] + TBD + water: 89.46 mg KOH / g polyol
   - Reaction medium [EmPyr][Br] + TBD + water: 67.23 mg KOH / g polyol

### Example 15: Depolymerization of a PU foam mass at 95°C for 6 h in a depolymerization reaction medium containing [Bmim][Cl] or [BmPyrr][Cl] as ionic liquid, TBD as superbase, and 1,2-isopropylideneglycerol (solketal) as nucleophilic agent.

In two 50 mL screw cap containers, 2 g of [Bmim][CI] or [BmPyrr][Cl] were added. Next, 0.4 g of TMG, 0.6 g of solketal, and 1 g of shredded polyurethane foam were added to each of them, being kept closed and under mechanical stirring for 6 h at 95°C. Then, 20 mL of water were added to each container to stop the reaction. The precipitated products were subjected to the same method of sample separation, drying and analysis described in Example 4, showing the following results:
▪ **Level Control 1 *- Solubility test.*** The addition of 20 mg of each of the dry polyols obtained over 2 mL of DMSO allowed the complete solubilization thereof, resulting in translucent solutions in all cases. This indicates that the depolymerization of the polyurethane foam mass has taken place.
▪ **Level Control 2 *- ATR-FTIR spectroscopy analysis*** (see Figure 14). Each of the dry polyols obtained was analyzed by ATR-FTIR, comparing their spectra (2, 3) with that of the original polyurethane foam mass (1). The characteristic band of the urethane bond (1700-1740 cm⁻¹) was not detected in any of the cases: reaction medium [Bmim][CI] + TBD + solketal (spectrum 2); reaction medium [BmPyrr][Cl] + TBD + solketal (spectrum 3). This confirms the correct depolymerization of the polyurethane foam mass according to the method of the invention.
▪ **Level Control 3 *- Hydroxyl value determination.*** Since the hydroxyl values obtained for the different solid fractions are much higher than that of the starting polyurethane foam mass, the correct depolymerization thereof according to these preferred embodiments of the method of the invention is again confirmed:
   - PU standard: 9.45 mg KOH / g polyol
   - Reaction medium [Bmim][CI] + TBD + solketal: 117.19 mg KOH / g polyol
   - Reaction medium [BmPyrr][Cl] + TBD + solketal: 65.71 mg KOH / g polyol

### Example 16: Rupture of the urethane bond of a mass of oxybis(ethane-2,1-diyl) bis(phenylcarbamate) in a reaction medium containing [Bmim][Cl] as ionic liquid, DBU, TBD, pyrrolidine, tert-butylamine or triethylamine as basic catalyst, and water as nucleophilic agent.

In five 50 mL screw cap containers, 1.334 g of [Bmim][CI] were added. Next, the corresponding superbase was added to each of them: 0.267 g DBU (pKₐ= 13.5); 0.249 g of TBD (pKₐ= 15.2); 0.148 g pyrrolidine (pKₐ= 11.3); 0.189 g tert-butylamine (pKₐ= 10.7); or 0.251 g triethylamine (pKₐ= 10.8). And lastly, 0.4 g of water and 0.05 g oxybis(ethane-2,1-diyl) bis(phenylcarbamate) were added, said containers being kept closed and under mechanical stirring for 4 h at 95°C. After that period has elapsed, 10 mL of chloroform and 10 mL of water were added to the reaction mixture. The phases were separated, and the organic phase was washed with water (2 x 10 mL). Finally, the organic phase was collected, dried with anhydrous MgSO₄ and the solvent was evaporated in a rotavapor. The products obtained were analyzed by ATR-FTIR (Figure 15a) and HPLC (Figure 15b).

As can be observed in spectra 2 and 3 of Figure 15a, in the reactions carried out in the presence of ionic liquid, superbase and nucleophilic agent, there is a complete disappearance of the carbonyl corresponding to the urethane bond, confirmed by the absence of a band at 1700-1740 cm⁻¹. However, this band is present when the ionic liquid and the nucleophilic agent are combined with bases with a pKₐ less than 12 (see spectra 4, 5 and 6 of Figure 15a). These results were also confirmed in the HPLC chromatograms (Figure 15b), where the peak corresponding to the urethane bond disappears in favor of the one associated with the rupture of the carbamate, and other new ones appear associated with the ionic species formed between the superbase (whether DBU or TBD) and the hydrolysis product (see chromatograms 2 and 3 vs. chromatograms 4-6 in Figure 15b).

### Example 17: Isolation and characterization of the product isolated after the rupture of the urethane bond of a mass of oxybis(ethane-2,1-diyl) bis(phenylcarbamate) in a reaction medium containing [Bmim][Cl] as ionic liquid, DBU as superbase, and water as nucleophilic agent.

After completing the depolymerization reaction described in Example 16 in which a reaction medium made up of [Bmim][CI], DBU and water is used, the major product obtained is isolated by means of chromatographic column by using silica gel as the stationary phase and a mixture of dichloromethane/methanol as the mobile phase (varying the ratio from 100/0 to 95/15 v/v). The pure product was analyzed by:
- **¹H-NMR** (300 MHz, CDCl₃): δ 7.88 (s, 2H) | 7.34 (dt, *J* = 8.8, 1.7 Hz, 4H) | 7.25 - 7.15 (m, 4H) | 7.00 - 6.83 (m, 2H) | 6.16 (t, *J* = 5.9 Hz, 2H) | 3.37 (t, *J* = 6.7 Hz, 4H) | 3.24 (dd, *J* = 10.3, 6.0 Hz, 4H) | 3.21 - 3.08 (m, 5H) | 2.56 - 2.37 (m, 4H) | 1.77 - 1.47 (m, 16H) (see Figure 16A);
- **¹³C-NMR** (101 MHz, CDCl₃): δ 176.64 | 156.28 | 139.74 | 128.86 | 122.26 | 119.26 | 49.67 | 45.65 | 37.18 | 36.88 | 29.83 | 28.38 | 28.21 | 23.32 (see Figure 16B);
- **ATR-FTIR:** *v* (cm⁻¹) = 3337 | 3336 | 3331 | 2930 | 1690 | 1613 | 1597 | 1548 | 1497 | 1440 | 1368 | 1353 | 1310 | 1227 | 1199 | 1176 | 1153 | 1112 | 1081 | 1031 | 978 | 909 | 853 | 753 | 726 | 693 | 644 (see Figure 16C), and
- **Mass spectroscopy:** m/z: [M+Na]⁺ calculated for [C₁₆H₂₃N₃O₂Na]⁺ = 312.1689; found: 312.1686.

### Example 18: HPLC method for monitoring the evolution of the transformation of the carbamate groups throughout the rupture of the urethane bond of oxybis(ethane-2,1-diyl) bis(phenylcarbamate).

The conditions of the HPLC-DAD method used were: C-18 column of 100 mm length, 0.5 mL/min flow, initial pressure 43-44 bar, column temperature 30°C, detector temperature 50°C, sample volume 10 µL, wavelength 220 nm, time 30 min, gradient acetonitrile (ACN)-water (5% ACN from 0 to 5 min, 5 to 20% ACN from 5 to 8 min, 20 to 60% ACN from 8 to 13 min, 60 to 95% ACN from 13 to 18 min, being maintained at 95% ACN from 18 to 23 min, then decreasing from 95 at 5% ACN from 23 to 25 min and being maintained at 5% ACN from 25 to 30 min). In these conditions, the presence of the urethane model oxybis(ethane-2,1-diyl) bis(phenylcarbamate)) was identified with the appearance of a signal at a retention time of 18.12 min (see chromatogram A in Figure 17b).

The sampling of the products analyzed in the different reactions with the urethane model oxybis(ethane-2,1-diyl) bis(phenylcarbamate) consisted of dissolving all the product obtained (according to the method described in Example 16) in 3 mL of MeOH, and then a 50 µL aliquot of said solution was taken and diluted in 1 mL of MeOH.

### Example 19: Rupture of the urethane bond of a mass of oxybis(ethane-2,1-diyl) bis(phenylcarbamate) in depolymerization reaction media containing [Bmim][Cl] as ionic liquid, different amounts of DBU as superbase, and water as nucleophilic agent.

1.334 g of [Bmim][Cl] were added to three 50 mL screw cap containers. Next, the corresponding amount of DBU (0.267 g for the study at 12.3 equivalents, 0.108 g for the study of 5 equivalents, 0 g for the study of the absence of DBU), 0.4 g of water and 0.05 g of oxybis(ethane-2,1-diyl) bis(phenylcarbamate) were added to each of them, said containers being kept closed and under mechanical stirring for 4 h at 95°C. After that period has elapsed, they are subjected to the same method of separation and drying described in Example 16.

The products isolated after the rupture of the urethane bond of oxybis(ethane-2,1-diyl) bis(phenylcarbamate) in each case were analyzed by ¹H-NMR (Figure 17a) and HPLC (Figure 17b). The results obtained again demonstrate that, in the absence of one of the elements of the reaction mixture, in this case the superbase, the urethane bond is not cleaved (compare spectrum E with C and D in Figures 17a and 17b), thus recovering the starting urethane intact.

### Example 20: Depolymerization of a PU foam mass at 95°C for 6 h in a depolymerization reaction medium containing [Bmim][Cl] as ionic liquid, DBU, TBD, pyrrolidine, tert-butylamine or triethylamine as basic catalyst, and water as nucleophilic agent.

2 g of [Bmim][CI] were added to five 50 mL screw cap containers. Next, 0.4 g of the corresponding basic catalyst (DBU, TBD, pyrrolidine, tert-butylamine or triethylamine), 0.6 g of water and 1 g of ground polyurethane foam were added to each of them, said containers being kept closed and under mechanical stirring for 6 h at 95°C. Then, 20 mL of water were added to each container to stop the reaction. The precipitated products were subjected to the same method of sample separation, drying and analysis described in Example 3, showing the following results:
▪ **Level Control 1 *- Solubility test.*** The addition of 20 mg of the dry polyols obtained using the superbase DBU or the superbase TBD as a basic catalyst over 2 mL of DMSO allowed the complete solubilization thereof, resulting in translucent solutions. In contrast, for the products obtained using a base, that is, pyrrolidine, tert-butylamine or triethylamine, as a basic catalyst, the addition of 20 mg of said products over 2 mL of DMSO resulted in an insoluble precipitate.
▪ **Level Control 2 *- ATR-FTIR spectroscopy analysis*** (see Figure 18). Each of the dry polyols obtained was analyzed by ATR-FTIR, comparing their spectra (2-6) with that of the original polyurethane foam mass (spectrum 1). The characteristic band of the urethane bond (1700-1740 cm⁻¹) was not detected in the cases where DBU (spectrum 2) or TBD (spectrum 3) was used as the superbase, but it was observed when pyrrolidine (spectrum 4), tert-butylamine (spectrum 5), or triethylamine (spectrum 6) was used.

These results confirm the same behavior observed in Example 16. Only the use of a reaction medium consisting of the combination of an ionic liquid, a superbase and a nucleophilic agent leads to the rupture of the urethane bond and, therefore, to the depolymerization of a polyurethane mass at temperatures less than 100°C.

### Example 21: Rupture of the urethane bond of a mass of oxybis(ethane-2,1-diyl) bis(phenylcarbamate) in a reaction medium containing [Bmim][Cl], [Bmim][AcO], [TBP][Br] or [BmPyr][Cl] as ionic liquid, DBU as superbase and water as nucleophilic agent.

In four 50 mL screw cap containers, 1.334 g of [Bmim][Cl], 1.666 g [Bmim][AcO], 2.5917 g [TBP][Br] or 1.4183 g [BmPyr][Cl] were added, respectively. Next, 0.267 g of DBU, 0.4 g of water, and 0.05 g of the urethane model oxybis(ethane-2,1-diyl) bis(phenylcarbamate) were added to each of them, being kept closed and under mechanical stirring for 4 h at 95°C. After that period has elapsed, they were subjected to the same method of separation and drying described in Example 16. The products obtained were analyzed by HPLC (see Figure 19).

This embodiment again exemplifies the need for the reaction medium to be made up of an ionic liquid, a superbase and a nucleophilic agent to achieve the cleavage of the urethane bond. Regardless of the type of ionic liquid used, evidence of the efficiency of said cleavage is shown by the complete disappearance of the peak associated with the urethane bond.

## Claims

1. A method for depolymerizing a polyurethane mass, wherein said method comprises performing the following steps:
a) mixing the polyurethane mass with a depolymerization reaction medium, wherein said depolymerization reaction medium comprises an ionic liquid, a basic catalyst and a nucleophilic agent; and
b) obtaining two immiscible fractions of the mixture obtained in step a):
- a first fraction, comprising at least a polyol mass; and
- a second fraction, comprising at least the ionic liquid and the basic catalyst;
and wherein said method is **characterized in that:**
- the depolymerization reaction medium comprises:
▪ an ionic liquid, in a proportion equal to or greater than 50% w/w with respect to the total weight of the depolymerization reaction medium;
▪ a superbase as a basic catalyst; and
▪ a nucleophilic agent in a proportion equal to or less than 15% w/w with respect to the total weight of the depolymerization reaction medium;
- step a) is carried out at a temperature between 50-100°C for a time period comprised between 2 minutes and 10 hours; and
- step b) comprises performing the following sub-steps:
i. adding a mass of a protic molecular solvent in an amount between 4 and 40 times more the weight of the depolymerization reaction medium to the mixture of step a);
ii. subjecting the mixture of step i) to a temperature between 20-60°C for at least 1 hour; and
iii. separating the fractions obtained.

2. The method according to the preceding claim, wherein the polyurethane mass comprises a thermoset polyurethane; or a polyurethane foam.

3. The method according to any of the preceding claims, wherein in step a) the polyurethane mass is mixed with the depolymerization reaction medium in a mass ratio comprised between 1:20 and 1:1; or between 1:15 and 1:3; or between 1:10 and 1:5.

4. The method according to any of the preceding claims, wherein step a) is carried out:
- at a temperature comprised between 80-100°C and/or for a time period comprised between 2 and 8 hours; or
- at a temperature comprised between 90-98°C and/or for a time period comprised between 3 and 7 hours.

5. The method according to any of the preceding claims, wherein step a) is carried out in a depolymerization reaction medium comprising an ionic liquid in a proportion between 50-85% w/w or between 65-80% w/w with respect to the total weight of the depolymerization reaction medium.

6. The method according to any of the preceding claims, wherein step a) is carried out in a depolymerization reaction medium comprising a nucleophilic agent in a proportion between 1-15% w/w or between 5-15% w/w with respect to the total weight of the depolymerization reaction medium.

7. The method according to any of the preceding claims, wherein in step b) a mass of a protic molecular solvent is added in an amount between 5 and 15 times more the weight of the depolymerization reaction medium.

8. The method according to any of the preceding claims, wherein step a) is carried out with a nucleophilic agent comprising water, an alcohol, an amine, or any possible combination thereof; or wherein the nucleophilic agent comprises at least 80% water.

9. The method according to any of the preceding claims, wherein sub-step i) is carried out with a protic molecular solvent comprising water, methanol, ethanol, isopropanol, or any possible combination thereof; or wherein the protic molecular solvent comprises at least 80% water.

10. The method according to any of the preceding claims, wherein step a) is carried out with an ionic liquid comprising:
- a cation selected from dialkylimidazolium, tetraalkylammonium, dialkylpiperidinium, alkylpyridinium, dialkylpyrrolidinium, and/or tetraalkylphosphonium, and/or
- an anion selected from fluoride, chloride, bromide, iodide, acetate, trifluoroacetate, formate, and/or carbonate,
or with an ionic liquid comprising 1-butyl-3-methylimidazolium acetate, 1-ethyl-3-methylimidazolium acetate, 1-butyl-3-methylpyridinium chloride, 1-butyl-3-methylpyridinium bromide, 1-butyl-3-methylpyridinium iodide, 1-butyl-1-methylpiperidinium chloride, 1-butyl-1-methylpiperidinium bromide, 1-butyl-1-methylpiperidinium iodide, 1-butyl-3-methylimidazolium chloride, 1-butyl-3-methylimidazolium bromide, 1-butyl-3-methylimidazolium iodide, 1-ethyl-3-methylimidazolium chloride, 1-ethyl-3-methylimidazolium bromide, 1-ethyl-3-methylimidazolium iodide, 1-butyl-4-methylpyridinium chloride, 1-butyl-3-methylimidazolium formate, 1-hexyl-3-methylimidazolium chloride, 1-hexyl-3-methylimidazolium bromide, 1-hexyl-3-methylimidazolium iodide, 1-octyl-3-methylimidazolium chloride, 1-octyl-3-methylimidazolium bromide, 1-octyl-3-methylimidazolium iodide, 1-allyl-3-methylimidazolium chloride, 1-butyl-3-methyl-imidazolium acetate, 1-butyl-3-methylimidazolium bromide, 1-ethyl-2-methylpyridinium bromide, N-ethylpyridinium bromide, 1-butyl-3-methyl-imidazolium chloride, 1-butyl-1-methylpyrrolidinium chloride, 1-butyl-1-methylpiperidinium iodide, tetrabutylphosphonium bromide, tetrabutylphosphonium chloride, 1-butyl-2-methylpyridinium chloride, 1-ethyl-1-methyl-piperidinium chloride, or any possible combination thereof.

11. The method according to any of the preceding claims, wherein the superbase comprises at least a bicyclic amidine, a bicyclic amidine derivative, a guanidine, a guanidine derivative, or any possible combination thereof; or wherein the superbase comprises 1,8-diazabicyclo [5.4.0] undec-7-ene (DBU), 1,5-diazabicyclo [4.3.0] non-5-ene (DBN), 1,5,7-triazabicyclo [4.4.0] dec-5-ene (TBD), tetramethylguanidine (TMG), or any possible combination thereof.

12. The method according to any of the preceding claims, comprising at least one of the following additional steps:
- reusing the first fraction in the synthesis of a new polyurethane; and/or
- reusing the second fraction in step a) of a new process for depolymerization of a polyurethane mass; and/or
- drying the first and/or second fraction until reducing the water content of said fraction/s to a value of less than 1% w/w.

13. The method according to the preceding claim, wherein the step of drying comprises a process of lyophilization, vacuum evaporation, heating, distillation, resin adsorption, or any possible combination thereof.

14. The method according to claims 12-13, previously comprising a step of treating the second fraction with a hydrophobic adsorbent, or with activated carbon or polystyrene resin.

15. The method according to any of the preceding claims, wherein sub-step iii) comprises a process of centrifugation, decantation, filtration, vacuum filtration, resin adsorption, or any possible combination thereof and/or wherein the method comprises an additional step of verification of the progression and completion of step a) and/or b).

## Patentansprüche

1. Verfahren zur Depolymerisation einer Polyurethanmasse, wobei das Verfahren die Durchführung der folgenden Schritte umfasst:
a) Mischen der Polyurethanmasse mit einem Depolymerisationsreaktionsmedium, wobei das Depolymerisationsreaktionsmedium eine ionische Flüssigkeit, einen basischen Katalysator und ein nukleophiles Mittel umfasst; und
b) Erhalten von zwei nicht mischbaren Fraktionen des in Schritt a) erhaltenen Gemisches:
- eine erste Fraktion, die mindestens eine Polyolmasse umfasst; und
- eine zweite Fraktion, die mindestens die ionische Flüssigkeit und den basischen Katalysator umfasst;
wobei das Verfahren **dadurch gekennzeichnet ist, dass:**
- das Depolymerisationsreaktionsmedium Folgendes umfasst:
▪ eine ionische Flüssigkeit in einem Anteil gleich oder größer als 50 Gew.-% in Bezug auf das Gesamtgewicht des Depolymerisationsreaktionsmediums;
▪ eine Superbase als basischen Katalysator; und
▪ ein nukleophiles Mittel in einem Anteil von gleich oder weniger als 15 Gew.- % in Bezug auf das Gesamtgewicht des Depolymerisationsreaktionsmediums;
- Schritt a) bei einer Temperatur zwischen 50-100 °C über eine Zeitperiode zwischen 2 Minuten und 10 Stunden durchgeführt wird; und
- Schritt b) die Durchführung der folgenden Teilschritte umfasst:
i. Hinzufügen einer Masse eines protischen molekularen Lösungsmittels in einer Menge zwischen dem 4- und 40-fachen des Gewichts des Depolymerisationsreaktionsmediums zum Gemisch aus Schritt a);
ii. Aussetzen des Gemisches aus Schritt i) einer Temperatur zwischen 20-60 °C für mindestens 1 Stunde; und
iii. Trennen der erhaltenen Fraktionen.

2. Verfahren nach dem vorhergehenden Anspruch, wobei die Polyurethanmasse ein duroplastisches Polyurethan oder einen Polyurethanschaum umfasst.

3. Verfahren nach einem der vorhergehenden Ansprüche, wobei in Schritt a) die Polyurethanmasse mit dem Depolymerisationsreaktionsmedium in einem Massenverhältnis zwischen 1:20 und 1:1; oder zwischen 1:15 und 1:3; oder zwischen 1:10 und 1:5 gemischt wird.

4. Verfahren nach einem der vorhergehenden Ansprüche, wobei Schritt a) wie folgt durchgeführt wird:
- bei einer Temperatur zwischen 80-100 °C und/oder über eine Zeitperiode zwischen 2 und 8 Stunden; oder
- bei einer Temperatur zwischen 90-98 °C und/oder über eine Zeitperiode zwischen 3 und 7 Stunden.

5. Verfahren nach einem der vorhergehenden Ansprüche, wobei Schritt a) in einem Depolymerisationsreaktionsmedium durchgeführt wird, das eine ionische Flüssigkeit in einem Anteil zwischen 50-85 Gew.-% oder zwischen 65-80 Gew.-%, in Bezug auf das Gesamtgewicht des Depolymerisationsreaktionsmediums, umfasst.

6. Verfahren nach einem der vorhergehenden Ansprüche, wobei Schritt a) in einem Depolymerisationsreaktionsmedium durchgeführt wird, das ein nukleophiles Mittel in einem Anteil zwischen 1-15 Gew.-% oder zwischen 5-15 Gew.-%, in Bezug auf das Gesamtgewicht des Depolymerisationsreaktionsmediums, umfasst.

7. Verfahren nach einem der vorhergehenden Ansprüche, wobei in Schritt b) ein protisches molekulares Lösungsmittel in einer Menge zwischen dem 5- und 15-fachen des Gewichts des Depolymerisationsreaktionsmediums hinzugefügt wird.

8. Verfahren nach einem der vorhergehenden Ansprüche, wobei Schritt a) mit einem nukleophilen Mittel durchgeführt wird, das Wasser, einen Alkohol, ein Amin oder eine beliebige Kombination davon umfasst; oder wobei das nukleophile Mittel mindestens 80 % Wasser umfasst.

9. Verfahren nach einem der vorhergehenden Ansprüche, wobei der Teilschritt i) mit einem protischen molekularen Lösungsmittel durchgeführt wird, das Wasser, Methanol, Ethanol, Isopropanol oder eine beliebige Kombination davon umfasst; oder wobei das protische molekulare Lösungsmittel mindestens 80 % Wasser umfasst.

10. Verfahren nach einem der vorhergehenden Ansprüche, wobei Schritt a) mit einer ionischen Flüssigkeit durchgeführt wird, umfassend:
- ein Kation, ausgewählt aus Dialkylimidazolium, Tetraalkylammonium, Dialkylpiperidinium, Alkylpyridinium, Dialkylpyrrolidinium und/oder Tetraalkylphosphonium, und/oder
- ein Anion, ausgewählt aus Fluorid, Chlorid, Bromid, Iodid, Acetat, Trifluoracetat, Formiat und/oder Carbonat, oder mit einer ionischen Flüssigkeit, umfassend 1-Butyl-3-methylimidazoliumacetat, 1-Ethyl-3-methylimidazoliumacetat, 1-Butyl-3-methylpyridiniumchlorid, 1-Butyl-3-methylpyridiniumbromid, 1-Butyl-3-methylpyridiniumiodid, 1-Butyl-1-methylpiperidiniumchlorid, 1-Butyl-1-methylpiperidiniumbromid, 1-Butyl-1-methylpiperidiniumiodid, 1-Butyl-3-methylimidazoliumchlorid, 1-Butyl-3-methylimidazoliumbromid, 1-Butyl-3-methylimidazoliumiodid, 1-Ethyl-3-methylimidazoliumchlorid, 1-Ethyl-3-methylimidazoliumbromid, 1-Ethyl-3-methylimidazoliumiodid, 1-Butyl-4-methylpyridiniumchlorid, 1-Butyl-3-methylimidazoliumformiat, 1-Hexyl-3-methylimidazoliumchlorid, 1-Hexyl-3-methylimidazoliumbromid, 1-Hexyl-3-methylimidazoliumiodid, 1-Octyl-3-methylimidazoliumchlorid, 1-Octyl-3-methylimidazoliumbromid, 1-Octyl-3-methylimidazoliumiodid, 1-Allyl-3-methylimidazoliumchlorid, 1-Butyl-3-methylimidazoliumacetat, 1-Butyl-3-methylimidazoliumbromid, 1-Ethyl-2-methylpyridiniumbromid, N-Ethylpyridiniumbromid, 1-Butyl-3-methylimidazoliumchlorid, 1-Butyl-1-methylpyrrolidiniumchlorid, 1-Butyl-1-methylpiperidiniumiodid, Tetrabutylphosphoniumbromid, Tetrabutylphosphoniumchlorid, 1-Butyl-2-methylpyridiniumchlorid, 1-Ethyl-1-methylpiperidiniumchlorid oder eine beliebige Kombination davon.

11. Verfahren nach einem der vorhergehenden Ansprüche, wobei die Superbase mindestens ein bicyclisches Amidin, ein Derivat eines bicyclischen Amidins, ein Guanidin, ein Guanidinderivat oder eine beliebige Kombination davon umfasst; oder wobei die Superbase 1,8-Diazabicyclo[5.4.0]undec-7-en (DBU), 1,5-Diazabicyclo[4.3.0]non-5-en (DBN), 1,5,7-Triazabicyclo[4.4.0]dec-5-en (TBD), Tetramethylguanidin (TMG) oder eine beliebige Kombination davon umfasst.

12. Verfahren nach einem der vorhergehenden Ansprüche, umfassend mindestens einen der folgenden zusätzlichen Schritte:
- Wiederverwendung der ersten Fraktion bei der Synthese eines neuen Polyurethans; und/oder
- Wiederverwendung der zweiten Fraktion in Schritt a) eines neuen Vorgangs zur Depolymerisation einer Polyurethanmasse; und/oder
- Trocknen der ersten und/oder zweiten Fraktion, bis der Wassergehalt der Fraktion(en) auf einen Wert von weniger als 1 Gew.-% reduziert ist.

13. Verfahren nach dem vorhergehenden Anspruch, wobei der Schritt des Trocknens einen Vorgang der Gefriertrocknung, Vakuumverdampfung, Erhitzung, Destillation, Harzadsorption oder eine beliebige Kombination davon umfasst.

14. Verfahren nach den Ansprüchen 12-13, vorab umfassend einen Schritt des Behandelns der zweiten Fraktion mit einem hydrophoben Adsorptionsmittel oder mit Aktivkohle oder Polystyrolharz.

15. Verfahren nach einem der vorhergehenden Ansprüche, wobei der Teilschritt iii) einen Vorgang der Zentrifugation, Dekantierung, Filtration, Vakuumfiltration, Harzadsorption oder eine beliebige Kombination davon umfasst und/oder wobei das Verfahren einen zusätzlichen Schritt zur Überprüfung des Fortschritts und des Abschlusses von Schritt a) und/oder b) umfasst.

## Revendications

1. Procédé de dépolymérisation d'une masse de polyuréthane, dans lequel ledit procédé comprend la réalisation des étapes suivantes:
a) mélanger la masse de polyuréthane avec un milieu de réaction de dépolymérisation, dans lequel ledit milieu de réaction de dépolymérisation comprend un liquide ionique, un catalyseur basique et un agent nucléophile; et
b) obtenir deux fractions immiscibles du mélange obtenu dans l'étape a):
- une première fraction, comprenant au moins une masse de polyol; et
- une deuxième fraction, comprenant au moins le liquide ionique et le catalyseur basique;
et dans lequel ledit procédé est **caractérisé en ce que:**
- le milieu de réaction de dépolymérisation comprend:
▪ un liquide ionique, dans une proportion égale ou supérieure à 50 % p/p par rapport au poids total du milieu de réaction de dépolymérisation;
▪ une superbase en tant que catalyseur basique; et
▪ un agent nucléophile dans une proportion égale ou inférieure à 15 % p/p par rapport au poids total du milieu de réaction de dépolymérisation;
- l'étape a) est réalisée à une température comprise entre 50-100 °C pendant une période de temps comprise entre 2 minutes et 10 heures; et
- l'étape b) comprend la réalisation des sous-étapes suivantes:
i. ajouter une masse d'un solvant moléculaire protique dans une quantité comprise entre 4 et 40 fois le poids du milieu de réaction de dépolymérisation au mélange de l'étape a);
ii. soumettre le mélange de l'étape i) à une température comprise entre 20-60 °C pendant au moins 1 heure; et
iii. séparer les fractions obtenues.

2. Procédé selon la revendication précédente, dans lequel la masse de polyuréthane comprend un polyuréthane thermodurcissable ; ou une mousse de polyuréthane.

3. Procédé selon l'une quelconque des revendications précédentes, dans lequel, dans l'étape a), la masse de polyuréthane est mélangée avec le milieu de réaction de dépolymérisation dans un rapport massique compris entre 1:20 et 1:1; ou entre 1:15 et 1:3; ou entre 1:10 et 1:5.

4. Procédé selon l'une quelconque des revendications précédentes, dans lequel l'étape a) est réalisée:
- à une température comprise entre 80-100 °C et/ou pendant une période de temps comprise entre 2 et 8 heures; ou
- à une température comprise entre 90-98 °C et/ou pendant une période de temps comprise entre 3 et 7 heures.

5. Procédé selon l'une quelconque des revendications précédentes, dans lequel l'étape a) est réalisée dans un milieu de réaction de dépolymérisation comprenant un liquide ionique dans une proportion comprise entre 50-85 % p/p ou entre 65-80 % p/p par rapport au poids total du milieu de réaction de dépolymérisation.

6. Procédé selon l'une quelconque des revendications précédentes, dans lequel l'étape a) est réalisée dans un milieu de réaction de dépolymérisation comprenant un agent nucléophile dans une proportion comprise entre 1-15 % p/p ou entre 5-15 % p/p par rapport au poids total du milieu de réaction de dépolymérisation.

7. Procédé selon l'une quelconque des revendications précédentes, dans lequel, dans l'étape b), une masse d'un solvant moléculaire protique est ajoutée dans une quantité comprise entre 5 et 15 fois le poids du milieu de réaction de dépolymérisation.

8. Procédé selon l'une quelconque des revendications précédentes, dans lequel l'étape a) est réalisée avec un agent nucléophile comprenant l'eau, un alcool, une amine, ou toute combinaison possible de ceux-ci ; ou dans lequel l'agent nucléophile comprend au moins 80 % d'eau.

9. Procédé selon l'une quelconque des revendications précédentes, dans lequel la sous-étape i) est réalisée avec un solvant moléculaire protique comprenant l'eau, le méthanol, l'éthanol, l'isopropanol ou toute combinaison possible de ceux-ci; ou dans lequel le solvant moléculaire protique comprend au moins 80 % d'eau.

10. Procédé selon l'une quelconque des revendications précédentes, dans lequel l'étape a) est réalisée avec un liquide ionique comprenant:
- un cation choisi parmi le dialkylimidazolium, le tétraalkylammonium, le dialkylpipéridinium, l'alkylpyridinium, le dialkylpyrrolidinium et/ou le tétraalkylphosphonium, et/ou
- un anion choisi parmi le fluorure, le chlorure, le bromure, l'iodure, l'acétate, le trifluoroacétate, le formiate, et/ou le carbonate,
ou avec un liquide ionique comprenant l'acétate de 1-butyl-3-méthylimidazolium, l'acétate de 1-éthyl-3-méthylimidazolium, le chlorure de 1-butyl-3-méthylpyridinium, le bromure de 1-butyl-3-méthylpyridinium, le iodure de 1-butyl-3-méthylpyridinium, le chlorure de 1-butyl-1-méthylpipéridinium, le bromure de 1-butyl-1-méthylpipéridinium, le iodure de 1-butyl-1-méthylpipéridinium, le chlorure de 1-butyl-3-méthylimidazolium, le bromure de 1-butyl-3-méthylimidazolium, le iodure de 1-butyl-3-méthylimidazolium, le chlorure de 1-éthyl-3-méthylimidazolium, le bromure de 1-éthyl-3-méthylimidazolium, le iodure de 1-éthyl-3-méthylimidazolium, le chlorure de 1-butyl-4-méthylpyridinium, le formiate de 1-butyl-3-méthylimidazolium, le chlorure de 1-hexyl-3-méthylimidazolium, le bromure de 1-hexyl-3-méthylimidazolium, le iodure de 1-hexyl-3-méthylimidazolium, le chlorure de 1-octyl-3-méthylimidazolium, le bromure de 1-octyl-3-méthylimidazolium, le iodure de 1-octyl-3-méthylimidazolium, le chlorure de 1-allyl-3-méthylimidazolium, l'acétate de 1-butyl-3-méthyl-imidazolium, le bromure de 1-butyl-3-méthyl-imidazolium, le bromure de 1-éthyl-2-méthylpyridinium, le bromure de N-éthylpyridinium, le chlorure de 1-butyl-3-méthyl-imidazolium, le chlorure de 1-butyl-1-méthylpyrrolidinium, le iodure de 1-butyl-1-méthylpipéridinium, le bromure de tétrabutylphosphonium, le chlorure de tétrabutylphosphonium, le chlorure de 1-butyl-2-méthylpyridinium, le chlorure de 1-éthyl-1-méthyl-pipéridinium, ou toute combinaison possible de ceux-ci.

11. Procédé selon l'une quelconque des revendications précédentes, dans lequel la superbase comprend au moins une amidine bicyclique, un dérivé d'amidine bicyclique, une guanidine, un dérivé de guanidine, ou toute combinaison possible de ceux-ci ; ou dans lequel la superbase comprend le 1,8-diazabicyclo[5.4.0]undéc-7-ène (DBU), le 1,5-diazabicyclo[4.3.0]non-5-ène (DBN), le 1,5,7-triazabicyclo[4.4.0]déc-5-ène (TBD), la tétraméthylguanidine (TMG), ou toute combinaison possible de ceux-ci.

12. Procédé selon l'une quelconque des revendications précédentes, comprenant au moins l'une des étapes additionnelles suivantes:
- réutiliser la première fraction dans la synthèse d'un nouveau polyuréthane; et/ou
- réutiliser la deuxième fraction dans l'étape a) d'un nouveau procédé de dépolymérisation d'une masse de polyuréthane; et/ou
- sécher la première et/ou deuxième fraction jusqu'à réduction de la teneur en eau de ladite/desdites fraction(s) jusqu'à une valeur inférieure à 1 % p/p.

13. Procédé selon la revendication précédente, dans lequel l'étape de séchage comprend un procédé de lyophilisation, d'évaporation sous vide, de chauffage, de distillation, d'adsorption dans une résine, ou toute combinaison possible de ceux-ci.

14. Procédé selon les revendications 12-13, comprenant préalablement une étape de traitement de la deuxième fraction avec un adsorbant hydrophobe, ou avec du charbon activé ou une résine de polystyrène.

15. Procédé selon l'une quelconque des revendications précédentes, dans lequel la sous-étape iii) comprend un procédé de centrifugation, décantation, filtration, filtration sous vide, adsorption dans une résine, ou toute combinaison possible de ceux-ci et/ou dans lequel le procédé comprend une étape additionnelle de vérification de la progression et de l'achèvement de l'étape a) et/ou b).
